# EUROPEAN PATENT APPLICATION

(11) **EP 4 725 967 A1**
(43) Date of publication of application: **15.04.2026**
(21) Application number: 24822702.7
(22) Date of filing: 12.06.2024
(51) Int. Cl.: C07K 16/28, A61K 47/68, A61K 39/395, A61K 45/00, A61P 35/00

(54) **ANTIBODY BINDING TO HUMAN CCR8 AND THE USE THEREOF**

(30) Priority: 12.06.2023 CN 202310693601
(71) Applicant: Nanjing Probio Biotech Co., Ltd., Nanjing, Jiangsu 211100 (CN)
(72) Inventor: LI, Zhongdao, Nanjing, Jiangsu 211100 (CN); CHEN, Li, Nanjing, Jiangsu 211100 (CN); ZHOU, Yi, Nanjing, Jiangsu 211100 (CN); LIANG, Yu, Nanjing, Jiangsu 211100 (CN)
(74) Representative: Synergy IP Group AG
(86) International application number: PCT/CN2024/098592
(87) International publication number: WO 2024/255753

(57) **Abstract**

The present application relates to a monoclonal antibody that specifically binds to CCR8 or an antigen-binding portion thereof. The present application also relates to a nucleic acid molecule encoding the antibody or the antigen-binding portion thereof; an expression vector, host cell and method for expressing the antibody or the antigen-binding portion thereof; and a therapeutic method using the antibody or the antigen-binding portion thereof, the nucleic acid molecule, the expression vector and/or the host cell.

## Description

### CROSS-REFERENCE TO RELATED APPLICATIONS

The present application claims priority to Chinese Patent Application No. 202310693601.4 filed on June 12, 2023, which is incorporated herein by reference in its entirety.

### TECHNICAL FIELD

The present application relates to an antibody or an antigen-binding portion thereof that specifically binds to CCR8, and use of the antibody or the antigen-binding portion in the treatment of diseases such as cancer.

### BACKGROUND

Chemokines can bind to chemokine receptors expressed on cells and guide cell migration through their own concentration distribution. The binding of chemokine receptors to chemokines can trigger signalling pathways, and the receptors undergo internalization and are re-expressed on the leading edge of migrating cells. Chemokines can be produced under homeostatic conditions, responsible for the daily migration of immune cells, or produced in response to inflammatory stimuli to recruit immune cells to infection sites or chronic inflammation sites (Moser B et al., (2004). Chemokines: multiple levels of leukocyte migration control. Trends Immunol. 25(2): 75-84; Zlotnik A et al., (2012) The chemokine superfamily revisited. Immunity. 36(5): 705-716).

T cells can be divided into different subsets according to the combination of chemokine receptors on the cell surface, such as regulatory T cells (Tregs) that regulate or suppress the immune system and play a role in maintaining self-tolerance and immune homeostasis, and cytotoxic CD8⁺ T cells that recognize and kill target cells. CCR8 is a chemokine receptor mainly expressed on Tregs, and ligands thereof are chemokines CCL1 and CCL18. The primary ligand CCL1 is mainly produced by activated monocytes, macrophages, and T lymphocytes, and can also be secreted by tumour cells, whereas the secondary ligand CCL18 can be secreted by tumour-associated macrophages (Moser B et al., (2022) Chemokine Receptor-Targeted Therapies: Special Case for CR8. Cancers 14(3): 511). Through the interaction between chemokines and chemokine receptors, Tregs migrate to inflammatory sites, triggering the CCR8 signalling pathway and balancing immunity and tolerance.

Tumour microenvironment (TME) is rich in various chemokines, and extensive infiltration of Tregs has been observed in the TME of a variety of tumour tissues, where these Tregs promote tumour growth by suppressing the anti-tumour immune response. The expression of CCR8 is mainly restricted to such tumour-infiltrating Tregs, and rarely occurs in Tregs from peripheral blood mononuclear cells. It has been reported that, compared to Tregs residing in normal tissues, CCR8 is upregulated only in tumour-infiltrating Tregs. In preclinical studies, it has been found that tumour-infiltrating Tregs highly express CCR8 in mouse models of intestinal cancer, melanoma, breast cancer, and urothelial carcinoma. Antibody therapy targeting CCR8 can selectively reduce CCR8⁺ tumour-infiltrating Tregs through mechanisms such as antibody-dependent cell-mediated cytotoxicity (ADCC), thereby triggering an anti-tumour immune response and significantly inhibiting tumour growth. In addition, CCR8 is also expressed on cutaneous memory T cells, which are considered the source of tumour cells in mycosis fungoides (MFs); depleting CCR8⁺ cells may inhibit the progression of T-cell lymphoma (Giustiniani J et al., (2022) CCR8 is a new therapeutic target in cutaneous T-cell lymphomas. Blood Adv. 6(11): 3507-3512). Therefore, CCR8⁺ Tregs residing in tumours and CCR8⁺ T cells residing in the skin may be suitable targets for cancer immunotherapy, and targeting CCR8 holds potential for cancer treatment.

However, CCR8, as a 7-transmembrane protein, is difficult to maintain its native configuration after purification. A variety of strategies have been used in the field to prepare antigens for animal immunization, including synthesizing partial extracellular domains or immunizing with DNA molecules. However, when peptides that fail to present the native conformation are used as antigens, the resulting antibodies may lack recognition capability for natural targets, while DNA immunization may be insufficient to elicit a robust immune response in animals. Therefore, immunization targeting this target is fraught with challenges, and novel methods are needed to enhance animal immunization and generate superior antibodies.

Reference to any document in the present application shall not be construed as an admission that such document constitutes prior art to the present application.

### SUMMARY

The inventors of the present application have screened for novel CCR8 antibodies by using a combination of DNA plasmids containing CCR8-encoding sequences, mRNA encoding CCR8, and/or cells overexpressing CCR8 in animal immunization.

Compared to antibodies in the prior art (such as BMS-986340 and GS-1811), the CCR8 antibodies of the present application have i) comparable or better CCR8-binding ability and binding specificity; ii) comparable or higher CCR8-CCL1 blocking ability; iii) comparable or higher ability to induce antibody-dependent cell-mediated cytotoxicity (ADCC) against CCR8⁺ cells; iv) comparable or higher internalization activity; and/or v) comparable or better *in vivo* anti-tumour effect.

Therefore, in a first aspect, the present application relates to an isolated monoclonal antibody (e.g., a murine, chimeric, or humanized antibody) or an antigen-binding portion thereof, which is capable of specifically binding to CCR8 (e.g., human or monkey CCR8), and may comprise: i) a heavy chain variable region, which may comprise VH CDR1, VH CDR2, and VH CDR3, wherein the VH CDR1, the VH CDR2, and the VH CDR3 may respectively comprise amino acid sequences as set forth in: (1) SEQ ID NOs: 1, 2, and 3; (2) SEQ ID NOs: 7, 8, and 9; (3) SEQ ID NOs: 13, 2, and 14; or (4) SEQ ID NOs: 18, 19, and 20; and/or ii) a light chain variable region, which may comprise VL CDR1, VL CDR2, and VL CDR3, wherein the VL CDR1, the VL CDR2, and the VL CDR3 may respectively comprise amino acid sequences as set forth in: (1) SEQ ID NOs: 4, 5, and 6; (2) SEQ ID NOs: 10, 11, and 12; (3) SEQ ID NOs: 15, 16, and 17; or (4) SEQ ID NOs: 21, 16, and 17. Further provided are variants of the antibody or the antigen-binding portion described above, which comprise up to about 3 amino acid substitutions (e.g., 1, 2, or 3 amino acid residue substitutions) in each CDR compared to the antibody or the antigen-binding portion described above. In some embodiments, the isolated monoclonal antibody or the antigen-binding portion thereof of the present application, which is capable of specifically binding to CCR8, comprises: i) a heavy chain variable region, comprising VH CDR1, VH CDR2, and VH CDR3, wherein the amino acid sequences of the VH CDR1, the VH CDR2, and the VH CDR3 may be respectively as set forth in: (1) SEQ ID NOs: 1, 2, and 3; (2) SEQ ID NOs: 7, 8, and 9; (3) SEQ ID NOs: 13, 2, and 14; or (4) SEQ ID NOs: 18, 19, and 20; and/or, ii) a light chain variable region, comprising VL CDR1, VL CDR2, and VL CDR3, wherein the amino acid sequences of the VL CDR1, the VL CDR2, and the VL CDR3 may be respectively as set forth in: (1) SEQ ID NOs: 4, 5, and 6; (2) SEQ ID NOs: 10, 11, and 12; (3) SEQ ID NOs: 15, 16, and 17; or (4) SEQ ID NOs: 21, 16, and 17.

The isolated monoclonal antibody or the antigen-binding portion thereof of the present application may comprise a heavy chain variable region and a light chain variable region, wherein the VH CDR1, the VH CDR2, the VH CDR3, the VL CDR1, the VL CDR2, and the VL CDR3 may respectively comprise amino acid sequences as set forth in: (1) SEQ ID NOs: 1, 2, 3, 4, 5, and 6; (2) SEQ ID NOs: 7, 8, 9, 10, 11, and 12; (3) SEQ ID NOs: 13, 2, 14, 15, 16, and 17; or (4) SEQ ID NOs: 18, 19, 20, 21, 16, and 17. In some embodiments, the VH CDR1, the VH CDR2, the VH CDR3, the VL CDR1, the VL CDR2, and the VL CDR3 may respectively comprise amino acid sequences as set forth in SEQ ID NOs: 1, 2, 3, 4, 5, and 6. In some embodiments, the VH CDR1, the VH CDR2, the VH CDR3, the VL CDR1, the VL CDR2, and the VL CDR3 may respectively comprise amino acid sequences as set forth in SEQ ID NOs: 7, 8, 9, 10, 11, and 12. Further provided are variants of the antibody or the antigen-binding portion described above, which comprise up to about 3 amino acid substitutions (e.g., 1, 2, or 3 amino acid residue substitutions) in each CDR compared to the antibody or the antigen-binding portion described above. In some embodiments, the isolated monoclonal antibody or the antigen-binding portion thereof of the present application may comprise a heavy chain variable region and a light chain variable region, wherein the amino acid sequences of the VH CDR1, the VH CDR2, the VH CDR3, the VL CDR1, the VL CDR2, and the VL CDR3 may be respectively as set forth in: (1) SEQ ID NOs: 1, 2, 3, 4, 5, and 6; (2) SEQ ID NOs: 7, 8, 9, 10, 11, and 12; (3) SEQ ID NOs: 13, 2, 14, 15, 16, and 17; or (4) SEQ ID NOs: 18, 19, 20, 21, 16, and 17.

The heavy chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 22, 24, 26, 27, 29, 31, 33, or 35.

The light chain variable region of the antibody or the antigen-binding portion thereof of the present application may comprise an amino acid sequence having at least 80%, 85%, 90%, 91%, 92%, 93%, 94%, 95%, 96%, 97%, 98%, 99%, or 100% sequence identity to SEQ ID NO: 23, 25, 28, 30, 32, 34, or 36.

The antibody or the antigen-binding portion thereof of the present application may comprise a heavy chain variable region and a light chain variable region, wherein the heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 22, 24, 26, 27, 29, 31, 33, or 35; and/or the light chain variable region may comprise an amino acid sequence having at least 80%, 85%, 90%, 91%, 92%, 93%, 94%, 95%, 96%, 97%, 98%, 99%, or 100% sequence identity to SEQ ID NO: 23, 25, 28, 30, 32, 34, or 36. In some embodiments, the heavy chain variable region may comprise an amino acid sequence as set forth in SEQ ID NO: 22, 24, 26, 27, 29, 31, 33, or 35; and the light chain variable region may comprise an amino acid sequence as set forth in SEQ ID NO: 23, 25, 28, 30, 32, 34, or 36. In some other embodiments, an amino acid sequence of the heavy chain variable region is as set forth in SEQ ID NO: 22, 24, 26, 27, 29, 31, 33, or 35; and an amino acid sequence of the light chain variable region is as set forth in SEQ ID NO: 23, 25, 28, 30, 32, 34, or 36.

The isolated monoclonal antibody or the antigen-binding portion thereof of the present application may comprise a heavy chain variable region and a light chain variable region, wherein the heavy chain variable region and the light chain variable region may respectively comprise amino acid sequences having at least 80%, 85%, 90%, 91%, 92%, 93%, 94%, 95%, 96%, 97%, 98%, 99%, or 100% sequence identity to: (1) SEQ ID NOs: 22 and 23; (2) SEQ ID NOs: 24 and 25; (3) SEQ ID NOs: 26 and 25; (4) SEQ ID NOs: 27 and 28; (5) SEQ ID NOs: 29 and 30; (6) SEQ ID NOs: 31 and 32; (7) SEQ ID NOs: 33 and 34; or (8) SEQ ID NOs: 35 and 36. In some embodiments, the heavy chain variable region and the light chain variable region may respectively comprise amino acid sequences as set forth in: (1) SEQ ID NOs: 22 and 23; (2) SEQ ID NOs: 24 and 25; (3) SEQ ID NOs: 26 and 25; (4) SEQ ID NOs: 27 and 28; (5) SEQ ID NOs: 29 and 30; (6) SEQ ID NOs: 31 and 32; (7) SEQ ID NOs: 33 and 34; or (8) SEQ ID NOs: 35 and 36. In some other embodiments, the amino acid sequences of the heavy chain variable region and the light chain variable region may be respectively as set forth in: (1) SEQ ID NOs: 22 and 23; (2) SEQ ID NOs: 24 and 25; (3) SEQ ID NOs: 26 and 25; (4) SEQ ID NOs: 27 and 28; (5) SEQ ID NOs: 29 and 30; (6) SEQ ID NOs: 31 and 32; (7) SEQ ID NOs: 33 and 34; or (8) SEQ ID NOs: 35 and 36.

In some embodiments, the isolated monoclonal antibody or the antigen-binding portion thereof of the present application may comprise a heavy chain variable region and a light chain variable region, wherein the VH CDR1, the VH CDR2, the VH CDR3, the VL CDR1, the VL CDR2, and the VL CDR3 may respectively comprise amino acid sequences as set forth in SEQ ID NOs: 1, 2, 3, 4, 5, and 6. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 22, 24, or 26. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 23 or 25. In some embodiments, the heavy chain variable region and the light chain variable region may respectively comprise amino acid sequences having at least 80%, 85%, 90%, 91%, 92%, 93%, 94%, 95%, 96%, 97%, 98%, 99%, or 100% sequence identity to: (1) SEQ ID NOs: 22 and 23; (2) SEQ ID NOs: 24 and 25; or (3) SEQ ID NOs: 26 and 25.

In some embodiments, the isolated monoclonal antibody or the antigen-binding portion thereof of the present application may comprise a heavy chain variable region and a light chain variable region, wherein the VH CDR1, the VH CDR2, the VH CDR3, the VL CDR1, the VL CDR2, and the VL CDR3 may respectively comprise amino acid sequences as set forth in SEQ ID NOs: 7, 8, 9, 10, 11, and 12. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 27 or 29. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 28 or 30. In some embodiments, the heavy chain variable region and the light chain variable region may respectively comprise amino acid sequences having at least 80%, 85%, 90%, 91%, 92%, 93%, 94%, 95%, 96%, 97%, 98%, 99%, or 100% sequence identity to: (1) SEQ ID NOs: 27 and 28; or (2) SEQ ID NOs: 29 and 30.

In some embodiments, the isolated monoclonal antibody or the antigen-binding portion thereof of the present application may comprise a heavy chain variable region and a light chain variable region, wherein the VH CDR1, the VH CDR2, the VH CDR3, the VL CDR1, the VL CDR2, and the VL CDR3 may respectively comprise amino acid sequences as set forth in SEQ ID NOs: 13, 2, 14, 15, 16, and 17. The heavy chain variable region may comprise an amino acid sequence having at least 80%, 85%, 90%, 91%, 92%, 93%, 94%, 95%, 96%, 97%, 98%, 99%, or 100% sequence identity to SEQ ID NO: 31 or 33. The light chain variable region may comprise an amino acid sequence having at least 80%, 85%, 90%, 91%, 92%, 93%, 94%, 95%, 96%, 97%, 98%, 99%, or 100% sequence identity to SEQ ID NO: 32 or 34. In some embodiments, the heavy chain variable region and the light chain variable region may respectively comprise amino acid sequences having at least 80%, 85%, 90%, 91%, 92%, 93%, 94%, 95%, 96%, 97%, 98%, 99%, or 100% sequence identity to: (1) SEQ ID NOs: 31 and 32; or (2) SEQ ID NOs: 33 and 34.

The present application further provides an isolated monoclonal antibody or an antigen-binding portion thereof, which can specifically bind to CCR8, and may comprise: i) a heavy chain variable region, which may comprise VH CDR1, VH CDR2, and VH CDR3, and ii) a light chain variable region, which may comprise VL CDR1, VL CDR2, and VL CDR3, wherein the VH CDR1, the VH CDR2, the VH CDR3, the VL CDR1, the VL CDR2, and the VL CDR3 may comprise VH CDR1, VH CDR2, VH CDR3, VL CDR1, VL CDR2, and VL CDR3 contained in the selected heavy chain variable region and light chain variable region, and the selected heavy chain variable region and light chain variable region may respectively comprise amino acid sequences as set forth in: (1) SEQ ID NOs: 22 and 23; (2) SEQ ID NOs: 24 and 25; (3) SEQ ID NOs: 26 and 25; (4) SEQ ID NOs: 27 and 28; (5) SEQ ID NOs: 29 and 30; (6) SEQ ID NOs: 31 and 32; (7) SEQ ID NOs: 33 and 34; or (8) SEQ ID NOs: 35 and 36.

The isolated monoclonal antibody or the antigen-binding portion thereof of the present application may comprise a heavy chain constant region and/or a light chain constant region, wherein the N-terminus of the heavy chain constant region is linked to the C-terminus of the heavy chain variable region, and the N-terminus of the light chain constant region is linked to the C-terminus of the light chain variable region. The heavy chain constant region may be an IgG, IgD, IgA, IgM, or IgE heavy chain constant region, preferably a heavy chain constant region that is natural or engineered to have an ability of binding to FcR and/or complement system proteins, or a functional fragment thereof, such as a fragment comprising the hinge region, CH2, and CH3 of the heavy chain constant region. In one embodiment, the heavy chain constant region is an IgG1 heavy chain constant region, such as a human IgG1 heavy chain constant region, comprising, for example, an amino acid sequence as set forth in SEQ ID NO: 37. The light chain constant region may be a κ or λ light chain constant region. In some embodiments, the light chain constant region may be a human κ light chain constant region, comprising, for example, an amino acid sequence as set forth in SEQ ID NO: 38.

In some embodiments, the antibody of the present application comprises or consists of two heavy chains and two light chains, wherein each of the heavy chains comprises the heavy chain constant region sequence, the heavy chain variable region sequence and/or the CDR sequences described above, and each of the light chains comprises the light chain constant region sequence, the light chain variable region sequence and/or the CDR sequences described above. In some embodiments, the antibody or the antigen-binding portion thereof of the present application may be Fab, F(ab')₂ fragment, Fv, scFv, (scFv)₂, or the like.

The antibody or the antigen-binding portion thereof of the present application may be, for example, murine, chimeric, or humanized.

The antibody or the antigen-binding portion thereof of the present application may be antagonistic.

The antibody or the antigen-binding portion thereof of the present application: i) may bind to human CCR8; ii) may bind to monkey CCR8; iii) block CCR8-CCL1 binding/interaction; iv) may be internalized by CCR8⁺ cells; v) may induce ADCC against CCR8⁺ cells (when the heavy chain constant region has FcR binding ability); and/or vi) have an *in vivo* anti-tumour effect.

The present application further provides an immunoconjugate comprising the antibody or the antigen-binding portion thereof of the present application, wherein the antibody or the antigen-binding portion thereof is linked to a therapeutic agent such as a cytotoxic molecule or an anti-cancer agent. The present application further provides a bispecific molecule comprising the antibody or the antigen-binding portion thereof of the present application, wherein the antibody or the antigen-binding portion thereof is linked to a second functional group, such as a second antibody, and the second functional group has a binding specificity different from that of the antibody or the antigen-binding portion thereof of the present application. In another aspect, the antibody or the antigen-binding portion thereof of the present application may be part of a chimeric antigen receptor (CAR) or a genetically engineered T-cell receptor (TCR). The present application further provides an immune cell comprising the CAR and/or TCR described above, including T cells, NK cells, and the like. The antibody or the antigen-binding portion thereof of the present application may also be encoded by or carried by an oncolytic virus.

The present application further includes a nucleic acid molecule encoding the antibody or the antigen-binding portion thereof, the immunoconjugate, the bispecific molecule, and/or the CAR/TCR of the present application. The present application may further provide an expression vector and a host cell. The expression vector may comprise the nucleic acid molecule of the present application. The host cell may comprise the expression vector of the present application or have the nucleic acid molecule of the present application integrated into the genome thereof.

The present application further provides a method for preparing the antibody or the antigen-binding portion thereof, the immunoconjugate, the bispecific molecule, or the CAR/TCR of the present application using the host cell of the present application, which comprises: (i) expressing the antibody or the antigen-binding portion thereof, the immunoconjugate, the bispecific molecule, or the CAR/TCR in the host cell; and (ii) isolating the antibody or the antigen-binding portion thereof, the immunoconjugate, the bispecific molecule, or the CAR/TCR from the host cell or a culture thereof.

The present application further provides a composition, which comprises the antibody or the antigen-binding portion thereof, the immunoconjugate, the bispecific molecule, the immune cell, the oncolytic virus, the nucleic acid molecule, the expression vector, or the host cell of the present application. In some embodiments, the composition is a pharmaceutical composition, which further comprises a pharmaceutically acceptable carrier. In some embodiments, the composition further comprises a PD-1 antibody.

In another aspect, the present application provides a method for treating or alleviating a CCR8-related disease in a subject, which comprises administering to the subject a therapeutically effective amount of the pharmaceutical composition of the present application. The CCR8-related disease may be a tumour, including solid tumours and haematological tumours. The solid tumours include, but are not limited to, non-small cell lung cancer (NSCLC), head and neck squamous cell carcinoma (HNSCC), gastric cancer, microsatellite-stable colorectal cancer, and cervical cancer. The haematological cancers may be T-cell lymphomas, including but not limited to cutaneous T-cell lymphoma. In some embodiments, the pharmaceutical composition of the present application may be administered with at least one additional therapeutic antibody, such as a PD-1 antibody. In another embodiment, the pharmaceutical composition of the present application may be administered with a cytokine (such as IL-2 and/or IL-21) or a co-stimulatory antibody (such as a CD137 antibody and/or a GITR antibody). In another embodiment, the antibody or the antigen-binding portion thereof of the present application may be administered with a chemotherapeutic agent, which may be a cytotoxic agent.

The present application further provides a method for reversing or alleviating immunosuppression in a subject, which comprises administering to the subject an effective amount of the pharmaceutical composition of the present application. In some embodiments, the method is used for reversing or alleviating immunosuppression in the tumour microenvironment, which comprises administering to a tumour site an effective amount of the pharmaceutical composition of the present application.

The present application further provides a method for enhancing an immune response in a subject, which comprises administering to the subject an effective amount of the pharmaceutical composition of the present application.

The present application further relates to use of the composition described above, particularly the pharmaceutical composition, in the preparation of a medicament for treating or alleviating a CCR8-related tumour, reversing or alleviating immunosuppression, or enhancing an immune response.

The antibody or the antigen-binding portion thereof of the present application may be further used in, for example, *in vitro* antigen detection and the like.

In another aspect, the present application provides a method for preparing a CCR8 antibody, which comprises: i) administering to an animal body a DNA molecule containing a CCR8-coding sequence, an RNA molecule encoding CCR8, and/or a cell capable of overexpressing CCR8; ii) collecting lymph node cells and/or spleen cells from the animal body; and iii) fusing the lymph node cells and/or the spleen cells with myeloma cells to produce hybridomas.

The DNA molecule containing the CCR8-coding sequence may be a DNA molecule that contains a CCR8-coding sequence and is capable of expressing a CCR8 protein in an animal body. The DNA molecule may be a DNA vector, such as a lentiviral vector or a plasmid. In some embodiments, the DNA molecule may be a vector containing an appropriate promoter and the CCR8-coding sequence. The promoter may be a constitutive promoter or an inducible promoter. In some embodiments, the promoter is a constitutive promoter. In some embodiments, the DNA molecule can be injected into an animal body, for example, subcutaneously or intraperitoneally, via, for example, a gene gun.

The RNA molecule encoding CCR8 may be an RNA molecule that contains a CCR8-coding sequence and is capable of expressing (translating) a CCR8 protein in an animal body. In some embodiments, the RNA molecule can express a human CCR8 protein in the animal body. The RNA molecule may be an mRNA molecule. In some embodiments, the RNA molecule may be a circular RNA molecule. In some embodiments, the RNA molecule may contain an appropriate promoter and the CCR8-coding sequence. The promoter may be a constitutive promoter or an inducible promoter. In some embodiments, the promoter may be a constitutive promoter. The RNA molecule can be directly injected into an animal body, for example, intraperitoneally. The RNA molecule can also be injected into an animal body with the assistance of lipid molecules (such as micelles, liposomes, lipid nanoparticles (LNPs)), virus-like particles (VLPs), polymer molecules (such as poly(lactic-co-glycolic acid) (PLGA), polyethyleneimine (PEI), polylysine (PLL), poly(β-amino ester) (PBAE), polymer nanoparticles), exosomes, and the like. In one embodiment, the RNA molecule is encapsulated by LNP.

The cell capable of overexpressing CCR8 may be a cell that expresses a CCR8 protein in an animal body. The cell may be a eukaryotic cell. In some embodiments, the cell can be injected into an animal body, for example, intraperitoneally.

In some embodiments, the method of the present application comprises: i) administering to an animal body an RNA molecule capable of expressing a CCR8 protein; ii) collecting lymph node cells and/or spleen cells from the animal body; and iii) fusing the lymph node cells and/or the spleen cells with myeloma cells to produce hybridomas. The RNA molecule can express a CCR8 protein in the animal body. In some embodiments, the RNA molecule can express a human CCR8 protein in the animal body.

All documents cited or referenced in the present application (including but not limited to all literatures, patents, and published patent applications cited herein) (documents cited in the present application), all documents cited or referenced in the documents cited in the present application, and any manufacturer's manuals, instructions, product specifications, and product sheets for any products referenced in the present application or any documents cited in the present application, are incorporated by reference into the present application and may be used in the practice of the present invention. More specifically, all reference documents are incorporated by reference into the present application to the same extent as if each reference document is incorporated by reference. Any Genbank sequence referenced herein is incorporated by reference into the present application.

It should be noted that in the present application, particularly in the claims, terms such as "comprise" and "include" may have the meanings as conferred by the Patent Law of the People's Republic of China; whereas terms such as "substantially consist of..." have the meanings as conferred by the Patent Law of the People's Republic of China, which, for example, permit the presence of elements not explicitly stated, but exclude elements that exist in the prior art or elements that affect the basic or novel properties of the present invention.

### BRIEF DESCRIPTION OF THE DRAWINGS

The following specific descriptions are provided by way of examples but are not intended to limit the present invention to the stated specific embodiments, and a better understanding thereof can be achieved in conjunction with the accompanying drawings.
FIGs. 1A and 1B show the binding ability of the chimeric antibodies 117G9F9, 125C1E6, 154G7C3 (1A), and 224E9A3 (1B) of the present application to HEK293 cells expressing human CCR8.
FIG. 2 shows the binding ability of the chimeric antibodies of the present application to HEK293 cells expressing monkey CCR8.
FIG. 3 shows the blocking ability of the chimeric antibodies of the present application against CCR8-CCL1 binding/interaction.
FIG. 4 shows the activity of the chimeric antibodies of the present application in inducing ADCC against CCR8⁺ cells.
FIGs. 5A-5C show the binding ability of the humanized antibodies of the present application to HEK293 cells expressing human CCR8 (5A), HEK293 cells expressing monkey CCR8 (5B), and parental HEK293 cells (5C).
FIGs. 6A and 6B show the activity of the humanized antibody 125C1E6 (6A), antibody 154G7C3, and antibody 117G9F9 (6B) of the present application in inducing ADCC against CCR8⁺ cells.
FIGs. 7A and 7B show the blocking ability of the humanized antibody 125C1E6 (7A, 7B), antibody 154G7C3, and antibody 117G9F9 (7B) of the present application against CCR8-CCL1 binding/interaction.
FIG. 8 shows the cellular internalization activity of the humanized antibodies of the present application.
FIGs. 9A-9C show the competitive epitope binding between the humanized antibody 125C1E6 of the present application and positive controls, in which in the experiment, the antibody 125C1E6 was added first, followed by the addition of BMS-986340, GS-1811-mIgG2a, or 125C1E6 (9A); BMS-986340 was added first, followed by the addition of 125C1E6-VH3-VL1 or BMS-986340 (9B); or GS-1811 was added first, followed by the addition of 125C1E6 (9C).

### DETAILED DESCRIPTION

For a better understanding of the present application, some terms are first defined. Other definitions are set forth throughout the detailed description.

The term "a" or "an" refers to one article or more than one article. For example, "an antibody" refers to one antibody or more than one antibody.

The term "CCR8" refers to CC chemokine receptor 8, which is a member of the G protein-coupled receptor (GPCR) family. The term includes variants, homologs, orthologs, and paralogs. For example, an antibody specific for human CCR8 may, under certain circumstances, cross-react with a CCR8 protein of another species, e.g., monkey. In other embodiments, an antibody specific for a human CCR8 protein may be fully specific for the human CCR8 protein without cross-reacting with proteins of other species or other types, or may cross-react with CCR8 proteins of some other species rather than all other species.

The term "human CCR8" refers to a CCR8 protein having a human amino acid sequence, such as the CCR8 protein having the amino acid sequence corresponding to GenBank Accession Number AAI07160.1 (Strausberg, R.L. et al., (2002) Generation and initial analysis of more than 15,000 full-length human and mouse cDNA sequences Proc. Natl. Acad. Sci. U.S.A. 99(26): 16899-16903). The term "monkey CCR8" refers to a CCR8 protein having a monkey amino acid sequence, such as the CCR8 protein having the amino acid sequence corresponding to GenBank Accession Number AFR51945.1 (Wang, L. et al., (2012), Submitted (26-JUL-2012) Department of Cellular Immunology and Pharmacology, Millennium Pharmaceuticals Inc.).

The term "antibody" herein is intended to include full-length IgG, IgA, IgD, IgE, and IgM antibodies and any antigen-binding fragment (i.e., antigen-binding portion) thereof. The full-length antibody is a glycoprotein comprising at least two heavy (H) chains and two light (L) chains, and the heavy chain and the light chain are linked by a disulfide bond. Each of the heavy chains is composed of a heavy chain variable region (abbreviated as V_{H} or VH) and a heavy chain constant region. The heavy chain constant region is composed of three domains: C_{H1}, C_{H2}, and C_{H3}. Each of the light chains is composed of a light chain variable region (abbreviated as V_{L} or VL) and a light chain constant region. The light chain constant region is composed of one domain C_{L}. The V_{H} and V_{L} regions may also be divided into hypervariable regions, known as complementarity determining regions (CDRs), which are separated by more conservative framework regions (FRs). The V_{H} and V_{L} are each composed of three CDRs and four FRs, arranged from the amino terminus to the carboxyl terminus in the following order: FR1, CDR1, FR2, CDR2, FR3, CDR3, FR4. The variable regions of the heavy chains and light chains comprise binding domains that interact with antigens. The constant region of an antibody can mediate the binding of immunoglobulins to host tissues or factors, including binding to various immune system cells (e.g., effector cells) and the first component (C1q) of the classical complement system. The "functional fragment" of the constant region of an antibody refers to a fragment of the constant region that retains certain desired functions, such as a fragment of the heavy chain constant region that retains FcR/complement system component-binding activity, e.g., an Fc fragment.

The term "antigen-binding portion" of an antibody (or abbreviated as an antibody portion) herein refers to one or more fragments of the antibody that retain the ability to specifically bind to an antigen (e.g., a CCR8 protein). It has been demonstrated that the antigen binding function of an antibody can be implemented by fragments of a full-length antibody. Examples of binding fragments included in the "antigen-binding portion" of an antibody include (i) a Fab fragment, that is, a monovalent fragment composed of V_{L}, V_{H}, C_{L}, and C_{H1}; (ii) an F(ab')₂ fragment, that is, a bivalent fragment comprising two Fab fragments linked by a disulfide bridge in the hinge region; (iii) an Fd fragment composed of V_{L} and C_{H1}; (iv) an Fv fragment composed of V_{L} and V_{H} from a single arm of an antibody; (v) a dAb fragment composed of V_{L} (Ward et al., (1989) Nature 341: 544-546); (vi) an isolated complementarity-determining region (CDR); and (vii) dAb-V_{L}, a fragment comprising a single variable domain and a heavy chain constant domain. In addition, although the two domains, V_{L} and V_{H}, of an Fv fragment are encoded by different genes, they can be linked by recombinant methods via a synthetic linker that brings the two into a single protein chain, in which the V_{L} and V_{H} regions are paired to form a monovalent molecule. These single chain antibodies are also intended to be included in the meaning of the term. These antibody fragments can be obtained by conventional techniques known to those skilled in the art, and the fragments can be functionally screened in the same manner as for intact antibodies.

The term "isolated antibody" as used herein refers to an antibody that is substantially free of other antibodies having different antigenic specificities. For example, an isolated antibody that specifically binds to a CCR8 protein is substantially free of antibodies that specifically bind to antigens other than the CCR8 protein. However, an isolated antibody that specifically binds to a human CCR8 protein may have cross-binding activity against other antigens, such as CCR8 proteins of other species. Furthermore, the isolated antibody is substantially free of other cellular materials and/or chemical substances.

The term "monoclonal antibody" or "mAb" or "monoclonal antibody composition" refers to an antibody molecule preparation consisting of a single type of molecule. The monoclonal antibody composition exhibits single binding specificity and affinity for a particular epitope.

The term "mouse antibody" refers to an antibody in which the framework regions and CDR regions in the variable region are derived from mouse germline immunoglobulin sequences. In addition, if the antibody comprises a constant region, the constant region is also derived from mouse germline immunoglobulin sequences. The mouse antibody of the present application may comprise amino acid residues not encoded by mouse germline immunoglobulin sequences, e.g., mutations introduced by *in vitro* random mutagenesis, site-directed mutagenesis, or *in vivo* somatic mutagenesis. However, the term "mouse antibody" does not include antibodies in which CDR sequences derived from other mammalian species are inserted into the mouse framework sequences.

The term "chimeric antibody" refers to an antibody that combines genetic substances of one species with genetic substances of another species. In particular, the chimeric antibody in the present application refers to an antibody obtained by combining genetic substances of non-human origin with genetic substances of human origin.

The term "humanized antibody" refers to an antibody that originates from a non-human species but whose protein sequence has been modified to increase its similarity to naturally occurring human antibodies.

The terms "antibody that recognizes an antigen" and "antibody specific for an antigen" are used interchangeably herein with the term "antibody that specifically binds to an antigen".

Herein, the terms "specifically recognize" or "specifically bind to" a target (e.g., human CCR8) mean that an antibody or an antigen-binding fragment is capable of distinguishing the target biomolecule from one or more reference molecules, and has binding affinity or binding activity for the target biomolecule that is, for example, 1-fold, 5-fold, or 10-fold higher than that for other reference molecules. Methods for determining specificity include, but are not limited to, Western blotting, ELISA, RIA, ECL, IRMA assays, and peptide scanning.

The term "substantially does not bind to" a protein or cell means not binding to the protein or cell at all, or not binding to the protein or cell with high affinity, i.e., the K_{D} for binding to the protein or cell is 1.0 × 10⁻⁶ M or greater, preferably 1.0 × 10⁻⁵ M or greater, more preferably 1.0 × 10⁻⁴ M or greater, or 1.0 × 10⁻³ M or greater, and more preferably 1.0 × 10⁻² M or greater.

The term "EC₅₀", also known as half-maximal effective concentration, refers to an antibody concentration that induces 50% of the maximum effect.

The term "IC₅₀" refers to half-maximal inhibitory concentration, i.e., the concentration of a drug or inhibitor required to inhibit a specified biological process by half.

The term "antibody-dependent cytotoxicity", "antibody-dependent cell-mediated cytotoxicity", or "ADCC" refers to a cell-mediated immune defence mechanism in which effector cells of the immune system actively lyse target cells whose cell membrane surface antigens are bound by antibodies (e.g., the CCR8 antibodies of the present application).

The term "subject" includes any human or non-human animal. The term "non-human animal" includes all vertebrates, such as mammals and non-mammals, e.g., non-human primates, sheep, dogs, cats, cattle, horses, chickens, amphibians, and reptiles, although mammals (e.g., non-human primates, sheep, dogs, cats, cattle, and horses) are preferred.

The term "therapeutically effective amount" refers to an amount of the antibodies of the present application sufficient to prevent or alleviate symptoms related to a disease or disorder (e.g., cancer). The therapeutically effective amount is related to the disease being treated, and the actual effective amount can be readily determined by those skilled in the art.

The term "sequence identity" herein refers to the percentage of nucleotides/amino acid residues in one sequence that are identical to those in a reference sequence after sequence alignment, where gaps are introduced into the sequence alignment if necessary to achieve the maximum percentage of sequence identity between the two sequences. Those skilled in the art can perform pairwise sequence alignment or multiple sequence alignment by various methods, e.g., using computer software, to determine the percentage of sequence identity between two or more nucleic acid or amino acid sequences. Examples of such computer software include ClustalOmega, T-coffee, Kalign, MAFFT, and the like.

The difficulty in preparing CCR8 antibodies lies in the fact that, as a 7-transmembrane protein, CCR8 is difficult to maintain its native conformation after purification. When peptides that fail to present the native conformation are used as antigens, the resulting antibodies may lack recognition capability for natural targets, while DNA immunization may be insufficient to elicit a robust immune response in animals.

The inventors of the present application have screened for novel CCR8 antibodies with superior properties compared to the prior art by using a combination of DNA plasmids containing CCR8-encoding sequences and cells overexpressing CCR8, or by administering mRNA encoding CCR8. The inventors of the present application have found that, for multi-transmembrane proteins, mRNA induces a higher antibody level than DNA molecules.

Therefore, in one aspect, the present application provides a novel method for immunizing animals, or a method for preparing antibodies via animal immunization, which comprises immunizing animals with RNA molecules (e.g., mRNA molecules) encoding the CCR8 protein.

Specifically, the present application provided a method for preparing a CCR8 antibody, which comprises: i) administering to an animal body a DNA molecule containing a CCR8-encoding sequence and capable of expressing a CCR8 protein in the animal body, an RNA molecule containing a CCR8-encoding sequence and capable of expressing (translating) a CCR8 protein in the animal body, and/or a cell capable of overexpressing a CCR8 protein in the animal body; ii) collecting lymph node cells and/or spleen cells of the animal body; and iii) fusing the lymph node cells and/or the spleen cells with myeloma cells to produce hybridomas.

In some embodiments, the method of the present application comprises: i) administering to an animal body a DNA molecule containing a CCR8-encoding sequence and capable of expressing a CCR8 protein in the animal body, and a cell capable of overexpressing a CCR8 protein in the animal body; ii) collecting lymph node cells and/or spleen cells of the animal body; and iii) fusing the lymph node cells and/or the spleen cells with myeloma cells to produce hybridomas.

In some embodiments, the method of the present application comprises: i) administering to an animal body an RNA molecule capable of expressing a CCR8 protein in the animal body; ii) collecting lymph node cells and/or spleen cells from the animal body; and iii) fusing the lymph node cells and/or the spleen cells with myeloma cells to produce hybridomas. In some embodiments, the RNA molecule can express a human CCR8 protein in the animal body. The RNA molecule may be an mRNA molecule, such as a circular RNA molecule. In some embodiments, the RNA molecule may be a vector containing an appropriate promoter and the CCR8-coding sequence. The promoter may be a constitutive promoter or an inducible promoter. In some embodiments, the promoter is a constitutive promoter. The RNA molecule can be directly injected into an animal body, for example, intraperitoneally. The RNA molecule can also be injected into an animal body with the assistance of lipid molecules (such as micelles, liposomes, lipid nanoparticles (LNPs)), virus-like particles (VLPs), polymer molecules (such as poly(lactic-co-glycolic acid) (PLGA), polyethyleneimine (PEI), polylysine (PLL), poly(β-amino ester) (PBAE), polymer nanoparticles), exosomes, and the like. In one embodiment, the RNA molecule is encapsulated by LNP.

Compared to the antibodies in the prior art (such as BMS-986340 and GS-1811), the CCR8 antibodies of the present application have i) comparable or better (human or monkey) CCR8-binding ability and binding specificity; ii) comparable or higher CCR8-CCL1 blocking ability; iii) comparable or higher ability to induce antibody-dependent cell-mediated cytotoxicity (ADCC) against CCR8⁺ cells; iv) comparable or higher internalization activity; and/or v) comparable or better *in vivo* anti-tumour effect.

Preferably, the antibody of the present application is a monoclonal antibody. In addition, the antibody may be, for example, a mouse, chimeric, or humanized monoclonal antibody.

The exemplary antibodies or the antigen-binding portions thereof of the present application are those whose structural and chemical properties are described below.

The sequences of the heavy chain variable regions and the light chain variable regions of the antibodies or the antigen-binding portions thereof of the present application are listed in Table 1 and Table 2. The CDRs of the heavy chain variable region and the light chain variable region are determined according to the Kabat numbering system, and the CDRs identified thereby are listed in Table 1. The CDRs of the heavy chain variable regions and the light chain variable regions of the antibodies or the antigen-binding portions thereof of the present application can also be determined according to the Chothia, IMGT, AbM, or Contact numbering system.

The antibodies or the antigen-binding portions thereof of the present application may comprise heavy chain constant regions, such as those that are natural or engineered to have FcR-binding ability, particularly high FcR-binding ability. In some embodiments, the heavy chain constant region may be an IgG1 constant region, such as a human IgG1 constant region comprising an amino acid sequence as set forth in SEQ ID NO: 37. The light chain constant region may be a κ constant region, such as a human κ constant region, which may comprise an amino acid sequence as set forth in SEQ ID NO: 38.

The V_{H} and/or V_{L} sequences (or CDR sequences) of other CCR8 antibodies that bind to human CCR8 can be "mixed and paired" with the V_{H} and/or V_{L} sequences (or CDR sequences) of the antibodies of the present application. Preferably, when the V_{H} and V_{L} (or CDRs therein) are mixed and paired, the V_{H} sequence in a particular V_{H}/V_{L} pair can be replaced by a structurally similar V_{H} sequence. Similarly, it is preferred that the V_{L} sequence in a particular V_{H}/V_{L} pair is replaced by a structurally similar V_{L} sequence.

Therefore, in one embodiment, the antibody or the antigen-binding portion thereof of the present application comprises:
(a) a heavy chain variable region comprising an amino acid sequence listed in Table 1 or Table 2; and
(b) a light chain variable region comprising an amino acid sequence listed in Table 1 or Table 2, or a V_{L} from another CCR8 antibody, wherein
the antibody specifically binds to human CCR8.

In another embodiment, the antibody or the antigen-binding portion thereof of the present application comprises:
(a) CDR1, CDR2 and CDR3 of a heavy chain variable region listed in Table 1 or Table 2; and
(b) CDR1, CDR2 and CDR3 of a light chain variable region listed in Table 1 or Table 2, or CDRs of a light chain variable region from another CCR8 antibody, wherein the antibody specifically binds to human CCR8.

In another embodiment, the antibody or the antigen-binding portion thereof of the present application comprises CDR2 of the heavy chain variable region of a CCR8 antibody, and CDRs from other antibodies that bind to human CCR8, for example, CDR1 and/or CDR3 of the heavy chain variable region, and/or CDR1, CDR2, and/or CDR3 of the light chain variable region from another CCR8 antibody.

Furthermore, it is well known in the art that the CDR3 domain, independent of the CDR1 and/or CDR2, can independently determine the binding specificity of antibodies for the same antigen, and can predict that multiple antibodies with the same binding specificity can be generated based on this CDR3 sequence. See, for example, Klimka et al., British J. of Cancer 83(2): 252-260 (2000); Beiboer et al, J. Mol. Biol. 296: 833-849 (2000); Rader et al., Proc. Natl. Acad. Sci U.S.A. 95: 8910-8915 (1998); Barbas et al, J. Am. Chem. Soc. 116: 2161-2162 (1994); Barbas et al., Proc. Natl. Acad. Sci. U.S.A. 92: 2529-2533 (1995); Ditzel et al., J Immunol. 157: 739-749 (1996).

In another embodiment, the antibody or the antigen-binding portion thereof of the present application comprises CDR2 of the heavy chain variable region of a CCR8 antibody, and at least CDR3 of the heavy chain and/or light chain variable region of the CCR8 antibody, or CDR3 of the heavy chain and/or light chain variable region of another CCR8 antibody, wherein the antibody or the antigen-binding portion thereof is capable of specifically binding to human CCR8. Preferably, these antibodies or antigen-binding portions thereof (a) compete for binding to CCR8; (b) retain functional properties; (c) bind to the same epitope; and/or (d) have binding affinity similar to that of the CCR8 antibodies or the antigen-binding portions thereof of the present application. In another embodiment, the antibody or the antigen-binding portion thereof may further comprise CDR2 of the light chain variable region of the CCR8 antibody or the antigen-binding portion thereof of the present application, or CDR2 of the light chain variable region of another CCR8 antibody, wherein the antibody or the antigen-binding portion thereof specifically binds to human CCR8. In another embodiment, the antibody of the present application may comprise CDR1 of the heavy/light chain variable region of the CCR8 antibody or the antigen-binding portion thereof of the present application, or CDR1 of the heavy and/or light chain variable region of another CCR8 antibody, wherein the antibody or the antigen-binding portion thereof specifically binds to human CCR8.

In another embodiment, the antibody or the antigen-binding portion thereof of the present application comprises heavy chain and/or light chain variable region sequences, or CDR1, CDR2 and CDR3 sequences, that have one or more conservative modifications relative to the CCR8 antibody or the antigen-binding portion thereof of the present application. It is known in the art that some conservative sequence modifications do not eliminate the antigen-binding activity. See, for example, Brummell et al., (1993) Biochem 32: 1180-8.

Therefore, in one embodiment, the antibody or the antigen-binding portion thereof comprises a heavy chain variable region and/or a light chain variable region each comprising CDR1, CDR2, and CDR3, wherein:
(a) CDR1 of the heavy chain variable region comprises a sequence listed in Table 1, and/or a conservatively modified version thereof; and/or
(b) CDR2 of the heavy chain variable region comprises a sequence listed in Table 1, and/or a conservatively modified version thereof; and/or
(c) CDR3 of the heavy chain variable region comprises a sequence listed in Table 1, and/or a conservatively modified version thereof; and/or
(d) CDR1, and/or CDR2, and/or CDR3 of the light chain variable region comprises a sequence listed in Table 1, and/or a conservatively modified version thereof; and
(e) the antibody or the antigen-binding portion thereof specifically binds to human CCR8.

The antibodies of the present application have one or more of the following functional characteristics, such as high affinity for and high specific binding to human CCR8, and corresponding enhanced ability to induce ADCC against CCR8-positive cells, such as CCR8-positive tumour cells.

In various embodiments, the antibody or the antigen-binding portion thereof may be, for example, murine, chimeric, or humanized.

The term "conservative sequence modification" as used herein refers to an amino acid modification that does not significantly affect or alter the binding properties of the antibody. Such conservative modifications include amino acid substitutions, additions and deletions. Modifications can be introduced into the antibody or the antigen-binding portion thereof of the present application by standard techniques known in the art, such as site-directed mutagenesis and PCR-mediated mutagenesis. Conservative amino acid substitutions are those in which an amino acid residue is replaced with an amino acid residue having a similar side chain. Groups of amino acid residues having similar side chains are known in the art. Such groups of amino acid residues include amino acids with basic side chains (e.g., lysine, arginine, and histidine), amino acids with acidic side chains (e.g., aspartic acid, and glutamic acid), amino acids with uncharged polar side chains (e.g., glycine, asparagine, glutamine, serine, threonine, tyrosine, cysteine, and tryptophan), amino acids with non-polar side chains (e.g., alanine, valine, leucine, isoleucine, proline, phenylalanine, and methionine), amino acids with β-branched side chains (e.g., threonine, valine, and isoleucine), and amino acids with aromatic side chains (e.g., tyrosine, phenylalanine, tryptophan, and histidine). Therefore, one or more amino acid residues in CDR regions of the antibody or the antigen-binding portion thereof of the present application can be replaced with other amino acid residues of the same side-chain group, and the resulting antibody can be tested for retained functions (i.e., the functions described above) using the functional assays described herein.

The antibody or the antigen-binding portion thereof of the present application can be prepared as a genetically modified antibody using an antibody having one or more V_{H}/V_{L} sequences of the CCR8 antibody or the antigen-binding portion thereof of the present application as a starting material. The antibody can be genetically modified by modifying one or more residues in one or both variable regions (i.e., V_{H} and/or V_{L}) (for example, in one or more CDR regions and/or one or more framework regions) to improve binding affinity and/or increase similarity to naturally occurring antibodies of certain species. For example, alternatively, the antibody can be genetically modified by modifying residues in the constant region, for example, to alter the effector function of the antibody.

In certain embodiments, CDR grafting can be used to genetically modify variable regions of an antibody. The antibody or the antigen-binding portion thereof mainly interacts with a target antigen through amino acid residues located in the six heavy and light chain complementarity determining regions (CDRs). For this reason, amino acid residues in CDRs are more diverse between individual antibodies than sequences outside the CDRs. Since CDR sequences are responsible for the major antibody-antigen interactions, recombinant antibodies simulating the properties of a particular natural antibody can be expressed by constructing expression vectors containing CDR sequences of the particular natural antibody grafted into the framework sequences of different antibodies with different properties.

Therefore, another embodiment of the present application relates to an isolated monoclonal antibody or an antigen-binding portion thereof, which comprises a heavy chain variable region and/or a light chain variable region, wherein the heavy chain variable region comprises CDR1, CDR2, and CDR3 having the sequences described above in the present application, and the light chain variable region comprises CDR1, CDR2, and CDR3 having the sequences described above in the present application. Although these antibodies comprise CDR sequences of the V_{H} and V_{L} of the monoclonal antibody of the present application, they may comprise different framework sequences.

Such framework sequences can be found in public DNA databases or public references including germline antibody gene sequences. For example, germline DNA sequences for human heavy and light chain variable region genes can be obtained from the Vbase human germline sequence database. As another embodiment, germline DNA sequences for human heavy and light chain variable region genes can be found in the Genbank database.

Antibody protein sequences are compared to protein sequences in the database by using one of the sequence similarity search methods known in the art as gap BLAST (Altschul et.al., (1997)).

Preferred framework sequences for the antibody or the antigen-binding portion thereof of the present application are those that are structurally similar to the framework sequences used in the antibody or the antigen-binding portion thereof of the present application. The V_{H} CDR1, CDR2, and CDR3 sequences can be grafted into framework regions that have the same sequence as the germline immunoglobulin gene from which the framework sequences are derived, or the CDR sequences can be grafted into framework regions containing one or more mutations compared to the germline sequence. For example, in some cases, it may be beneficial to mutate residues in the framework regions to maintain or enhance the antigen-binding ability of the antibody.

Another class of variable region modifications involves mutating amino acid residues in CDR1, CDR2, and/or CDR3 regions of V_{L} and/or V_{L} to improve one or more binding properties (e.g., affinity) of the antibody of interest. Mutations can be introduced by point mutations or PCR-mediated mutations, and the effect of the mutations on antibody binding or other functional properties can be evaluated through *in vitro* or *in vivo* assays known in the art. Preferably, conservative modifications known in the art are introduced. The mutation may be an amino acid substitution, addition, or deletion, and is preferably an amino acid substitution. In addition, generally no more than one, two, three, four, or five residues in the CDR regions are altered.

In addition, in another embodiment, the present application provides an isolated CCR8 monoclonal antibody or an antigen-binding portion thereof, which comprises a heavy chain variable region and a light chain variable region, wherein the heavy chain variable region and the light chain variable region comprise: (a) a V_{H} CDR1 region, comprising the sequence of the present application, or an amino acid sequence with one, two, three, four, or five amino acid substitutions, deletions, or additions; (b) a V_{H} CDR2 region, comprising the sequence of the present application, or an amino acid sequence with one, two, three, four, or five amino acid substitutions, deletions, or additions; (c) a V_{H} CDR3 region, comprising the sequence of the present application, or an amino acid sequence with one, two, three, four, or five amino acid substitutions, deletions, or additions; (d) a V_{L} CDR1 region, comprising the sequence of the present application, or an amino acid sequence with one, two, three, four, or five amino acid substitutions, deletions, or additions; (e) a V_{L} CDR2 region, comprising the sequence of the present application, or an amino acid sequence with one, two, three, four, or five amino acid substitutions, deletions, or additions; and (f) a V_{L} CDR3 region, comprising the sequence of the present application, or an amino acid sequence with one, two, three, four, or five amino acid substitutions, deletions, or additions.

The genetically engineered antibodies of the present application include those in which genetic modifications are made to the framework residues of V_{L} and/or V_{L} to, for example, alter antibody properties. Framework modifications include mutating one or more residues in the framework regions, or even in one or more CDR regions, to eliminate T-cell epitopes, thereby reducing the potential immunogenicity that an antibody may induce. This method is also known as "deimmunization" and is described in more detail in U.S. Patent Application No. 20030153043.

In addition, as an alternative to modifications in the framework or CDR regions, the antibodies of the present application can be genetically engineered to include genetic modifications in the Fc region, typically to alter one or more functional properties of the antibodies, such as serum half-life, complement fixation, Fc receptor binding, and/or antibody-dependent cellular cytotoxicity. In addition, the antibodies of the present application can be chemically modified (for example, one or more chemical functional groups can be attached to the antibodies) or modified to alter their glycosylation, so as to change one or more functional properties of the antibodies.

In one embodiment, the hinge region of C_{H1} is modified to alter, for example, to increase or decrease, the number of cysteine residues in the hinge region. This method is further described in U.S. Patent No. 5,677,425. The cysteine residues in the C_{H1} hinge region are altered, for example, to facilitate the assembly of heavy and light chains or to increase/decrease the stability of the antibody.

In another embodiment, the Fc hinge region of the antibody is mutated to increase or decrease the biological half-life of the antibody. More specifically, one or more amino acid mutations are introduced into the C_{H2}-C_{H3} linking region of the Fc hinge fragment such that the antibody has reduced SpA binding relative to natural Fc-hinge domain SpA binding. This method is described in more detail in U.S. Patent No. 6,165,745.

In another embodiment, the glycosylation of the antibody is modified. For example, deglycosylated antibodies (i.e., antibodies that lack glycosylation) can be prepared. Glycosylation can be altered to, for example, increase the affinity of the antibody for an antigen. Such glycosylation modifications can be achieved, for example, by altering one or more glycosylation sites in the sequence of the antibody. For example, one or more amino acid substitutions can be made to eliminate one or more variable region framework glycosylation sites, thereby eliminating glycosylation at these sites. Such deglycosylation may increase the affinity of the antibody for the antigen. See, for example, U.S. Patent Nos. 5,714,350 and 6,350,861.

In addition, antibodies with altered glycosylation patterns can be prepared, such as low-fucosylated antibodies with a reduced amount of fucose residues, or antibodies with an increased bisecting GlcNac structure. The altered glycosylation patterns have been demonstrated to increase the ADCC activity of the antibodies. Such glycosylation modifications can be performed, for example, by expressing the antibodies in a host cell with an altered glycosylation system. Cells with an altered glycosylation system are known in the art, including but not limited to an Slc35c1 gene knockout cell line, an FUT8 knockout cell line, a mutant CHO cell line Lec13, a rat hybridoma cell line YB2/0, a cell line containing small interfering RNAs specifically targeting the FUT8 gene, and a cell line co-expressing β-1,4-N-acetylglucosaminyltransferase III and Golgi α-mannosidase II. These cells can be used as host cells for expressing the recombinant antibodies of the present application to prepare antibodies with altered glycosylation.

Another modification of the antibodies herein is pegylation (PEGylation). The antibodies can be PEGylated, for example, to increase the biological (e.g., serum) half-life of the antibodies. To PEGylate an antibody, the antibody or the fragment thereof is typically reacted with polyethylene glycol (PEG), such as a reactive ester or aldehyde derivative of PEG, under conditions that allow one or more PEG groups to be attached to the antibody or the antibody fragment. Preferably, PEGylation is performed by an acylation reaction or an alkylation reaction with a reactive PEG molecule (or similar reactive water-soluble polymers). The term "polyethylene glycol" as used herein includes any form of PEG used to derivatize other proteins, such as (C₁-C₁₀) alkoxy- or aryloxy-polyethylene glycol or polyethylene glycol maleimide. In certain embodiments, the antibody to be PEGylated is a deglycosylated antibody. Methods for PEGylating proteins are known in the art and can be applied to the antibodies of the present application. See, for example, EPO 154 316 and EP 0 401 384.

The antibodies or the antigen-binding portions thereof of the present application can be characterized by their various physical properties, such that their classes are detected and/or distinguished.

For example, the antibodies or the antigen-binding portions thereof may contain one or more glycosylation sites in the light chain or heavy chain variable region. These glycosylation sites may induce increased antibody immunogenicity, or cause altered antibody pK values due to changed antigen binding. Glycosylation is known to occur in motifs containing N-X-S/T sequences. In some cases, it is preferred that the CCR8 antibody or the antigen-binding portion thereof does not contain variable region glycosylation. This can be achieved by selecting antibodies that do not contain glycosylation motifs in variable regions or by mutating residues in the glycosylation region.

In preferred embodiments, the antibody or the antigen-binding portion thereof does not contain an asparagine isomerization site. Deamidation of asparagine may occur at the N-G or D-G sequence, creating isoaspartic acid residues that introduce knobs into the polypeptide chain and reduce its stability (isoaspartic acid effect).

Each antibody or antigen-binding portion thereof will have a unique isoelectric point (pI), which substantially falls within the pH range of 6-9.5. The pI of IgG1 antibodies is typically within the pH range of 7-9.5, whereas the pI of IgG4 antibodies is substantially within the pH range of 6-8. It is speculated that antibodies with pI beyond the normal range may have some unfolding structures and be unstable under *in vivo* conditions. Therefore, it is preferred that the pI value of the CCR8 antibody falls within the normal range. This can be achieved by selecting antibodies with pI within the normal range or by mutating uncharged surface residues.

The monoclonal antibodies of the present application can be prepared using the somatic hybridization (hybridoma) technique in Kohler and Milstein (1975) Nature 256: 495. Other methods for preparing the monoclonal antibodies include the single B-cell antibody preparation technique and the phage display technique. Methods for preparing the chimeric or humanized antibodies are also well known in the art.

The antibodies or the antigen-binding portions thereof of the present application can also be produced in host cell transfectomas using, for example, recombinant DNA technology in combination with gene transfection methods (e.g., Morrison, S. (1985) Science 229: 1202). In one embodiment, DNA encoding partial or full-length light and heavy chains obtained by standard molecular biology techniques is inserted into one or more expression vectors such that the genes are operably linked to transcriptional and translational regulatory sequences. In this case, the term "operably linked" refers to the linkage of the antibody genes into the vector such that the transcriptional and translational control sequences within the vector perform their intended function of regulating the transcription and translation of the antibody genes.

The term "regulatory sequence" includes promoters, enhancers, and other expression control elements (e.g., polyadenylation signals) that control the transcription or translation of the antibody genes. Preferred regulatory sequences for expression in mammalian host cells include viral elements that direct high-level protein expression in mammalian cells, such as promoters and/or enhancers derived from cytomegalovirus (CMV), simian virus 40 (SV40), adenovirus (e.g., the adenovirus major late promoter (AdMLP)), and polyomavirus. Alternatively, non-viral regulatory sequences such as ubiquitin promoters or β-globin promoters can be used. Additionally, the regulatory elements are composed of sequences of different origins. For example, the SRα promoter system comprises the sequence from the SV40 early promoter and the long terminal repeat of the human T-cell leukaemia virus type I. The expression vector and expression control sequences are selected to be compatible with the expression host cell used.

The antibody light chain gene and the antibody heavy chain gene can be inserted into the same expression vector or different expression vectors. In preferred embodiments, variable regions are inserted into an expression vector that has encoded the heavy chain constant region and the light chain constant region of the desired subtype to construct a full-length antibody gene, such that the V_{H} is operably linked to the C_{H} in the vector and the V_{L} is operably linked to the C_{L} in the vector. Alternatively, the recombinant expression vector can encode a signal peptide that facilitates the secretion of antibody chains from the host cell. The antibody chain genes can be cloned into a vector such that the signal peptide is linked to the amino termini of the antibody chain genes in the reading frame. The signal peptide may be an immunoglobulin signal peptide or a heterologous signal peptide (i.e., a signal peptide from a non-immunoglobulin protein).

In addition to the antibody chain genes and regulatory sequences, the recombinant expression vectors of the present application can carry other sequences, such as sequences that regulate the replication of the vector in host cells (e.g., an origin of replication) and selectable marker genes. The selectable marker genes can be used to select a host cell into which the vector has been introduced. For example, the selectable marker genes generally confer drug resistance, e.g., G418, hygromycin, or methotrexate resistance, on the host cell into which the vector has been introduced. Preferred selectable marker genes include a dihydrofolate reductase (DHFR) (for methotrexate selection/amplification in dhfr host cells), and a neo gene (for G418 selection).

For expression of the light and heavy chains, the expression vectors encoding the heavy and light chains are transfected into the host cell by standard techniques. The term "transfection" in various forms includes a variety of techniques commonly used to introduce exogenous DNA into prokaryotic or eukaryotic host cells, e.g., electroporation, calcium phosphate precipitation, DEAE-dextrose transfection, and the like. Although expressing the antibodies or the antigen-binding portions thereof of the present application in prokaryotic or eukaryotic host cells is theoretically feasible, expressing the antibodies in eukaryotic cells is preferred, and expressing the antibodies in mammalian host cells is most preferred. This is because eukaryotic cells, particularly mammalian cells, are more likely to assemble and secrete properly folded and immunologically active antibodies than prokaryotic cells.

Preferred mammalian host cells for expressing the recombinant antibodies of the present application include an Slc35C1 gene knockout cell line, an FUT8 knockout cell line, a mutant CHO cell line Lec13, a rat hybridoma cell line YB2/0, a cell line containing small interfering RNAs specifically targeting the FUT8 gene, a cell line co-expressing β-1,4-N-acetylglucosaminyltransferase III and Golgi α-mannosidase II, Chinese hamster ovary (CHO cells) (including dhfr-CHO cells administered with a DHFR selectable marker), NSO myeloma cells, COS cells, and SP2 cells. When a recombinant expression vector encoding an antibody gene is introduced into a mammalian host cell, the antibody is prepared by culturing the host cell for a period of time sufficient to allow the expression of the antibody in the host cell, or preferably, sufficient to allow the secretion of the antibody into the medium in which the host cell grows. The antibody or the antigen-binding portion thereof can be recovered from the medium using protein purification methods.

In another aspect, the present disclosure provides a nucleic acid molecule encoding the heavy chain/light chain variable regions or CDRs of the antibody or the antigen-binding portion thereof of the present application. The nucleic acid may be present in an intact cell, in a cell lysate, or in a partially purified or substantially pure form. The nucleic acid is "isolated" or "substantially pure" when purified from other cellular components or other contaminants, such as other cellular nucleic acids or proteins, by standard techniques. The nucleic acid of the present application may be, for example, DNA or RNA, and may or may not contain an intron sequence. In preferred embodiments, the nucleic acid is a cDNA molecule.

The nucleic acid of the present application can be obtained using standard molecular biology techniques. For antibodies expressed by hybridomas (e.g., hybridomas prepared from transgenic mice carrying human immunoglobulin genes), cDNAs encoding the light and heavy chains of the antibodies prepared from the hybridomas can be obtained by standard PCR amplification or cDNA cloning techniques. For antibodies obtained from an immunoglobulin gene library (e.g., using phage display technique), nucleic acids encoding such antibodies can be collected from the gene library.

The preferred nucleic acid molecules of the present application include those encoding the V_{H} and V_{L} sequences or CDRs of the CCR8 monoclonal antibody. Once the DNA fragments encoding V_{H} and V_{L} are obtained, operations such as conversion of the variable region genes to full-length antibody chain genes, Fab fragment genes, or scFv genes can be further performed on these DNA fragments by standard recombinant DNA techniques. In these operations, the DNA fragment encoding V_{H} or V_{L} is operably linked to another DNA fragment encoding another protein, such as an antibody constant region or a flexible linker. The term "operably linked" means that two DNA fragments are linked together such that the amino acid sequences encoded by the two DNA fragments are both in the reading frame.

Isolated DNA encoding the V_{H} region can be converted into a full-length heavy chain gene by operably linking DNA encoding V_{H} to another DNA molecule encoding the heavy chain constant regions (C_{H1}, C_{H2}, and C_{H3}). The sequence of the human heavy chain constant region gene is known in the art, and DNA fragments comprising these regions can be obtained by standard PCR amplification. The heavy chain constant region may be an IgG1, IgG2, IgG3, IgG4, IgA, IgE, IgM, or IgD constant region, but is preferably an IgG1 constant region. For the Fab fragment heavy chain genes, DNA encoding the V_{H} region can be operably linked to another DNA molecule encoding only the heavy chain C_{H1} constant region.

Isolated DNA encoding the V_{L} region can be converted into a full-length light chain gene by operably linking DNA encoding V_{L} to another DNA molecule encoding the light chain constant region C_{L}. The sequences of human light chain constant region genes are known in the art, and DNA fragments comprising these regions can be obtained by standard PCR amplification. In preferred embodiments, the light chain constant region may be a κ or λ constant region.

To create an scFv gene, DNA fragments encoding V_{H} and V_{L} can be operably linked to another fragment encoding a flexible linker, such that the V_{H} and V_{L} sequences can be expressed as a continuous single-chain protein, with the V_{H} and V_{L} regions linked by the flexible linker.

The antibody or the antigen-binding portion thereof of the present application can be conjugated to a therapeutic agent to form an immunoconjugate, such as an antibody-drug conjugate (ADC). Suitable therapeutic agents include cytotoxic molecules, alkylating agents, DNA minor groove binding molecules, DNA intercalators, DNA cross-linking agents, histone deacetylase inhibitors, nuclear export inhibitors, proteasome inhibitors, inhibitors of topoisomerase I or II, heat shock protein inhibitors, tyrosine kinase inhibitors, antibiotics, and antimitotic agents. In ADCs, the antibody and the therapeutic agent can be cross-linked via a linker that is cleavable, such as a peptide linker, a disulfide linker, or a hydrazone linker. More preferably, the linker is a peptide linker, such as Val-Cit, Ala-Val, Val-Ala-Val, Lys-Lys, Ala-Asn-Val, Val-Leu-Lys, Ala-Ala-Asn, Cit-Cit, Val-Lys, Lys, Cit, Ser, or Glu. ADCs can be prepared as described in U.S. Patent Nos. 7,087,600, 6,989,452, and 7,129,261; PCT Publication Nos. WO 02/096910, WO 07/038,658, WO 07/051,081, WO 07/059,404, WO 08/083,312, and WO 08/103,693; and U.S. Patent Publication Nos. 20060024317, 20060004081, and 20060247295. In the present application, since the CCR8 antibody can specifically bind to and internalize into CCR8⁺ cells, particularly CCR8⁺ Treg cells, it can be conjugated to a cytotoxic molecule, such that the cytotoxic molecule can specifically damage CCR8⁺ Treg cells, thereby achieving the purpose of killing CCR8⁺ Treg cells. In particular, the cytotoxic molecule can enter CCR8⁺ Treg cells through antibody-mediated internalization. The cytotoxic molecule may be any small-molecule compound or protein molecule that causes damage to target cells, such as a tubulin polymerization inhibitor and a DNA-damaging agent.

In another aspect, the present application relates to a bispecific molecule comprising the antibody or the antigen-binding portion thereof of the present application linked to at least one additional functional molecule, such as another peptide or protein (e.g., another antibody or receptor ligand), to generate a bispecific molecule that binds to at least two different binding sites or target molecules. The term "bispecific molecule" includes molecules having three or more specificities.

The bispecific molecule may be present in a variety of forms and sizes. At one end of the size spectrum, the bispecific molecules remain in the conventional antibody format, except that they have two binding arms with different types of specificity instead of having two binding arms with the same specificity. At the other extreme end are bispecific molecules composed of two single-chain antibody fragments (scFvs) linked via a peptide chain, called Bs(scFv)₂ constructs. Medium-sized bispecific molecules comprise two different F(ab) fragments linked via a peptide linker. These and other forms of bispecific molecules can be prepared by genetic engineering, somatic hybridization, or chemical methods.

The present application further provides a chimeric antigen receptor comprising a CCR8 single-chain antibody scFv, wherein the scFv comprises the heavy chain and light chain CDRs, or the heavy chain and light chain variable regions, described in the present application.

The CCR8 chimeric antigen receptor may comprise: (a) an extracellular antigen-binding domain containing CCR8 scFv; (b) a transmembrane domain; and (c) an intracellular signal transduction domain.

The present application further provides an immune cell, such as a T cell or NK cell, which comprises the chimeric antigen receptor of the present application.

Oncolytic viruses preferentially infect and kill cancer cells. The antibody or the antigen-binding portion thereof of the present application can be used in combination with oncolytic viruses. In addition, an oncolytic virus encoding the antibody or the antigen-binding portion thereof of the present application can be introduced into a subject.

In another aspect, the present application provides a composition, which comprises the antibody or the antigen-binding portion thereof, the nucleic acid molecule, the expression vector, the host cell, immunoconjugate, the chimeric antigen receptor, the immune cell, the bispecific antibody, and/or the oncolytic virus of the present application. In some embodiments, the composition is a pharmaceutical composition, which further comprises a pharmaceutically acceptable carrier. The composition may optionally comprise one or more additional pharmaceutically active ingredients, such as another anti-tumour antibody or immune-enhancing antibody, or non-antibody anti-tumour agent or immune enhancer. The pharmaceutical composition of the present application can be used in combination with, for example, another anti-cancer agent or another immune enhancer.

The pharmaceutical composition may comprise any number of excipients. Excipients that may be used include carriers, surfactants, thickening or emulsifying agents, solid binders, dispersing or suspending agents, solubilizers, colorants, flavouring agents, coatings, disintegrants, lubricants, sweeteners, preservatives, isotonic agents, and combinations thereof. The selection and use of suitable excipients are taught in Gennaro, ed., Remington: The Science and Practice of Pharmacy, 20th Ed. (Lippincott Williams & Wilkins 2003).

Preferably, the pharmaceutical composition is suitable for intravenous, intramuscular, subcutaneous, parenteral, spinal, or epidermal (e.g., by injection or bolus injection). Depending on the route of administration, the active ingredient can be encapsulated in a material to protect it from acids and other natural conditions that may inactivate it. "Parenteral administration" refers to a mode that is different from enteral administration and topical administration and that is generally performed by injection, including but not limited to, intravenous, intramuscular, intra-arterial, intramembranous, intracapsular, intraorbital, intracardiac, intradermal, intraperitoneal, transtracheal, subcutaneous, subcuticular, intra-articular, subcapsular, subarachnoid, intraspinal, epidural and intrasternal injection and bolus injection. Alternatively, the antibody of the present application can be administered by a non-parenteral route, such as topical, epidermal, or mucosal administration, such as intranasal, oral, vaginal, rectal, sublingual, or topical administration.

The pharmaceutical compositions may be in the form of sterile aqueous solutions or dispersions. They may also be formulated in microemulsions, liposomes, or other ordered structures suitable for high concentrations of drugs.

The amount of active ingredient that is formulated together with a carrier material into a single dosage form will vary with a subject treated and a particular mode of administration, and is substantially the amount of the composition that produces a therapeutic effect. The amount is, by percentage, about 0.01% to about 99% of the active ingredient in combination with a pharmaceutically acceptable carrier.

The administration regimen is adjusted to provide the best desired response (e.g., therapeutic response). For example, a rapid perfusion agent may be administered, multiple divided doses may be administered over time, or the dose may be reduced or increased in proportion to the criticality of the treatment situation. It is particularly advantageous to formulate parenteral compositions in dosage units featuring ease of administration and uniformity of dosage. Dosage unit refers to physically separate units that are suitable for single administration to a treated subject; each unit contains a predetermined amount of the active ingredient calculated to produce the desired therapeutic effect in combination with the pharmaceutical carrier. Alternatively, the antibody can be administered in a sustained-release formulation, in which case the frequency of administration required is reduced.

For the administration of the antibody, the dose may be about 0.001-100 mg/kg body weight of a host. An exemplary treatment regimen involves administration once a week.

A "therapeutically effective amount" of the pharmaceutical composition of the present application results in a reduction in the severity of disease symptoms and an increase in the frequency and duration of asymptomatic periods. For example, for treatment of a subject with a tumour, a "therapeutically effective amount" preferably inhibits tumour growth by at least about 20%, more preferably by at least about 40%, even more preferably by at least about 60%, and more preferably by at least about 80%, relative to an untreated subject. A therapeutically effective amount of a therapeutic antibody can reduce tumour size, or alleviate a symptom in a subject, which may be a human or another mammal.

The pharmaceutical composition may be a sustained-release agent, including implants and microencapsulated delivery systems. Biodegradable and biocompatible polymers such as ethylene-vinyl acetate, polyanhydrides, polyglycolic acid, collagen, polyorthoesters, and polylactic acid may be used. See, for example, Sustained and Controlled Release Drug Delivery Systems, J. R. Robinson, ed., Marcel Dekker, Inc., New York, 1978.

The pharmaceutical composition can be administered via medical devices, such as (1) needle-free subcutaneous injection devices (e.g., U.S. Patent Nos. 5,399,163, 5,383,851, 5,312,335, 5,064,413, 4,941,880, 4,790,824, and 4,596,556); (2) microinfusion pumps (U.S. Patent No. 4,487,603); (3) transdermal administration devices (U.S. Patent No. 4,486,194); (4) bolus injection devices (U.S. Patent Nos. 4,447,233 and 4,447,224); and (5) osmotic devices (U.S. Patent Nos. 4,439,196 and 4,475,196).

In certain embodiments, the components of the composition of the present application can be formulated to ensure appropriate *in vivo* distribution. For example, to ensure that the therapeutic antibody or the antigen-binding portion thereof of the present application crosses the blood-brain barrier, the antibody can be formulated in liposomes, which may additionally contain targeting functional groups to enhance selective delivery to particular cells or organs. See, for example, U.S. Patent Nos. 4,522,811, 5,374,548, 5,416,016, and 5,399,331.

The present application also relates to *in vivo* gene therapy, in which a nucleic acid molecule encoding the antibody or the antigen-binding portion thereof, the immunoconjugate, the bispecific molecule or the like of the present application is directly introduced into a subject. For example, the nucleic acid sequence encoding the antibody or the antigen-binding portion of the present application is introduced into target cells via local injection through a nucleic acid construct with or without a suitable delivery vector, such as an adeno-associated virus vector. Other alternative viral vectors include, but are not limited to, retroviral, adenoviral, herpes simplex viral, and papillomaviral vectors. The *in vivo* physical transfer of viral vectors can be achieved via local injection, liposome-mediated transfer, direct injection (naked DNA), or particle bombardment (gene gun) of the desired nucleic acid construct or other suitable delivery vectors containing the desired nucleic acid sequence.

The pharmaceutical composition of the present application has various *in vitro* and *in vivo* applications, including, for example, the treatment of cancer, or more generally, the enhancement of immunity in patients with diseases such as cancer. The pharmaceutical composition can be administered to a human subject to, for example, inhibit tumour growth *in vivo.*

In view of the ability of the pharmaceutical composition of the present application to inhibit the proliferation and survival of tumour cells, the present application provides a method for inhibiting the growth of tumour cells in a subject, which comprises administering to the subject the pharmaceutical composition of the present application, thereby inhibiting tumour growth in the subject. Non-limiting examples of tumours that can be treated by the composition of the present application include, but are not limited to, solid tumours and haematological tumours. The solid tumours include, but are not limited to, non-small cell lung cancer (NSCLC), head and neck squamous cell carcinoma (HNSCC), gastric cancer, microsatellite-stable colorectal cancer, cervical cancer, and the like, whether primary or metastatic. The haematological cancers may be T-cell lymphomas, including but not limited to cutaneous T-cell lymphoma. In addition, refractory or relapsed malignant tumours may also be treatable with the pharmaceutical composition of the present application.

The present application provides a combination therapy of the pharmaceutical composition of the present application administered with one or more additional antibodies or non-antibody therapeutic agents, wherein the combination therapy can effectively inhibit tumour growth in a subject. In one embodiment, the present application provides a method for inhibiting tumour growth in a subject, which comprises administering to the subject the pharmaceutical composition of the present application and one or more additional antibodies, such as a PD-1 antibody. In certain embodiments, the subject is a human. In another aspect, the present application provides a method for treating cancer, wherein the pharmaceutical composition of the present application is administered with a chemotherapeutic agent, which may be a cytotoxic agent. Other therapies that may be used in combination with the pharmaceutical composition of the present application include, but are not limited to, administration of an immunogenic agent, administration of interleukin 2 (IL-2), radiotherapy, surgery, or hormone deprivation.

The composition of the present application can also be used to reverse or alleviate immunosuppression in a subject. In particular, the present application further provides a method for reversing or alleviating immunosuppression in a subject, which comprises administering to the subject an effective amount of the pharmaceutical composition of the present application. In some embodiments, the method is used for reversing or alleviating immunosuppression in the tumour microenvironment, which comprises administering to a tumour site an effective amount of the pharmaceutical composition of the present application.

The composition of the present application can also be used to enhance an immune response. In particular, the present application provides a method for enhancing an immune response in a subject, which comprises administering to the subject an effective amount of the pharmaceutical composition of the present application.

The combination of therapeutic agents discussed herein can be administered simultaneously as a single composition in a pharmaceutically acceptable carrier, or administered simultaneously as separate compositions, wherein each agent is in a pharmaceutically acceptable carrier. In another embodiment, the combination of therapeutic agents can be administered sequentially.

Furthermore, if the combination therapy is administered multiple times and the agents are administered sequentially, the sequence in the sequential administration at each time point can be reversed or maintained, and the sequential administration can be combined with simultaneous administration or any combination thereof.

The aspects and embodiments of the present application will be discussed with reference to the accompanying drawings and the following examples. Other aspects and embodiments will be apparent to those skilled in the art. All literatures described herein are incorporated herein by reference in their entireties. Although the present application has been described in conjunction with exemplary embodiments, many equivalent modifications and variations will be apparent to those skilled in the art upon the presentation of the present application. Therefore, the exemplary embodiments of the present application are illustrative rather than limiting. Various changes can be made to the described embodiments without departing from the spirit and scope of the present application.

### Examples

The following examples are given for the purpose of illustration only and are not intended to limit the present invention in any way.

### Example 1. Production of anti-human CCR8 monoclonal antibodies (mAbs)

To produce antibodies against human CCR8, wild-type female mice including BALB/C, A/J, or SJL mice were immunized with a variety of antigens, including DNA/mRNA encoding human CCR8 and cell lines overexpressing human CCR8. Specifically, the antigens described above were used alone or in combination for immunization. For example, the DNA plasmid encoding CCR8 (GenScript) and the CHO-K1 cell line overexpressing human CCR8 (CHO-K1/hu-CCR8, Cat #: RD00915, GenScript) were used together for immunization. Every two weeks, gold particles coated with the DNA plasmid (6 µg) were injected subcutaneously or intraperitoneally into the mice three times via the Helios gene gun system (Bio-Rad), followed by intraperitoneal (i.p.) injection of CHO-K1/hu-CCR8 cells (5 × 10⁶ cells per mouse). Alternatively, the mice were injected with 20/40 µg of mRNA LNPs (TheraRNA) three times every two weeks.

In addition, to evaluate the serum titer of immunized animals and screen/identify CCR8-specific antibodies, the CCR8-encoding genes of humans and monkeys were synthesized and inserted into the pLVX-Puro vector, which was then subjected to lentiviral packaging and used to infect HEK293 cells. Subsequently, screening was performed using puromycin to prepare HEK293 cell line-based stable cell lines overexpressing human and monkey CCR8, namely HEK293/hu-CCR8 cells (Cat #: RD00953, GenScript) and HEK293/cyno CCR8 cells (Cat #: RD01063, GenScript).

After evaluating the serum titer of the immunized mice, the mice with the highest human CCR8 antibody titer were selected. Lymph nodes and/or spleens were collected from these mice to isolate lymph node cells and/or spleen cells, which were then fused with myeloma cells to produce hybridomas. Specifically, the isolated lymph node cells and/or spleen cells were mixed with the mouse myeloma cell line SP2/0 at a ratio of 3:1, followed by electrofusion to produce fused cells. Subsequently, the fused cells were placed in a 96-well plate and incubated in a selective medium containing hypoxanthine-aminopterin-thymidine (HAT) for two weeks to produce hybridomas. Anti-human CCR8 antibodies were screened from the supernatant of each hybridoma well by flow cytometry using HEK293/hu-CCR8 cells. Then, positive hybridomas were subcloned by limiting dilution to obtain single clones.

The ability of antibodies expressed in monoclonal supernatants to bind to HEK293 cells overexpressing human/monkey CCR8 was analysed using flow cytometry. First, HEK293 cells overexpressing human/monkey CCR8, namely HEK293/hu-CCR8 and HEK293/cyno CCR8, were cultured and harvested, and then incubated with monoclonal supernatants containing anti-CCR8 antibodies. Then, the samples were analysed using a flow cytometer (BD Canto II). Two antibodies, BMS-986340 (prepared according to the disclosure in WO 2021194942 A1) and GS-1811 (prepared according to the disclosure in WO 2021163064 A2), were used as positive control antibodies, whereas unengineered parental HEK293 cells were used as negative control cells for the FACS binding assay.

The primary screening results showed that the antibodies produced by monoclonal clones 154G7C3 and 117G9F9 in the present application only bound to cells expressing human CCR8, whereas the antibodies produced by monoclonal clones 125C1E6 and 224E9A3 exhibited binding affinity for both HEK293 cells expressing human CCR8 and those expressing monkey CCR8. The clones 125C1E6, 154G7C3, 117G9F9, and 224E9A3 were sequenced, and unique clones were selected for subsequent development.

### Example 2. Preparation of chimeric antibodies

The coding sequences of the murine heavy chain variable region and light chain variable region from the selected and sequenced clones were fused to the N-terminus of the coding sequences of the human IgG1 heavy chain constant region and human κ light chain constant region, respectively. These fused sequences were then cloned into the expression plasmid pTT5, downstream of the signal peptide, for secretory expression. The amino acid sequences of the heavy chain and light chain variable regions are listed in Table 1, and the amino acid sequences of the human IgG1 heavy chain constant region and human κ light chain constant region are set forth in SEQ ID NO: 37 and 38, respectively.

**Table 1. Heavy chain and light chain variable regions and CDR information of the monoclonal antibodies of the present application**

| |
|---|
| 125C1E6 |
| Heavy chain variable region |
| VH CDR1: AYAMN (SEQ ID NO: 1) |
| VH CDR2: RIRSKSNYYATYYADSVKD (SEQ ID NO: 2) |
| VH CDR3: QGYFGTRESRRYFDV (SEQ ID NO: 3) |
| EVQLVESGGGLVQPKGSLKLSCAASGFNFNAYAMNWVRQAPGKGLEW VARIRSKSNYYATYYADSVKDRFTISRDDSESMLYLQMNNLKTEDTAM YYCVRQGYFGTRESRRYFDVWGTGTTVTVSS (SEQ ID NO: 22) |
| Light chain variable region |
| VL CDR1: KASESVGSYVS (SEQ ID NO: 4) |
| VL CDR2: GASTRYT (SEQ ID NO: 5) |
| VL CDR3: GQNYIYPLT (SEQ ID NO: 6) |
| NIVMTQSPKSMSMSVGERVTLSCKASESVGSYVSWYQQKPGQSPKLLIY GASTRYTGVPDRFTGSGSAKDFTLTISSVQAEDPADYHCGQNYIYPLTFG AGTKLELK (SEQ ID NO: 23) |
| 154G7C3 |
| Heavy chain variable region |
| VH CDR1: DFEMH (SEQ ID NO: 7) |
| VH CDR2: AFDPETGGTAYNQKFKG (SEQ ID NO: 8) |
| VH CDR3: RLRRHFDS (SEQ ID NO: 9) |
| QVQLQQSGAELVRPGASVTLSCKTSGYTFTDFEMHWVKQTPVHGLEWI GAFDPETGGTAYNQKFKGKAILTADKSSSTAYMELRSLTSEDSAVYYCT GRLRRHFDSWGQGTTLTVSS (SEQ ID NO: 27) |
| Light chain variable region |
| VL CDR1: RSSQSLVHSYGNTYLH (SEQ ID NO: 10) |
| VL CDR2: KVSNRFS (SEQ ID NO: 11) |
| VL CDR3: SQSTHVPFT (SEQ ID NO: 12) |
| DVVMTQTPLSLPVSLGDQASISCRSSQSLVHSYGNTYLHWYLQKPGQSP KLLIYKVSNRFSGVPDRFSGSGSGTDFTLKISRVEAEDLGVYFCSQSTHV PFTFGSGTKLEIK (SEQ ID NO: 28) |
| 117G9F9 |
| Heavy chain variable region |
| VH CDR1: TYAMN (SEQ ID NO: 13) |
| VH CDR2: RIRSKSNYYATYYADSVKD (SEQ ID NO: 2) |
| VH CDR3: GGNSYGSSYFDY (SEQ ID NO: 14) |
| EVQLVESGGGLVQPKGSLKLSCAASGFSFNTYAMNWVRQAPGKGLEW VARIRSKSNYYATYYADSVKDRFTISRDDSESMLYLQMNNLKTEDTAM YYCVRGGNSYGSSYFDYWGQGTTLTVSS (SEQ ID NO: 31) |
| Light chain variable region |
| VL CDR1: RSSKSLLHRNGNTYLY (SEQ ID NO: 15) |
| VL CDR2: RMSNLAS (SEQ ID NO: 16) |
| VL CDR3: MQHLEYPFT (SEQ ID NO: 17) |
| DIVMTQAAPSVPVTPGESVSISCRSSKSLLHRNGNTYLYWFLQRPGQSPQ LLIYRMSNLASGVPDRFSGSGSGTAFTLRISRLEAEDVGVYYCMQHLEY PFTFGAGTKLELK (SEQ ID NO: 32) |
| 224E9A3 |
| Heavy chain variable region |
| VH CDR1: TYAMY (SEQ ID NO: 18) |
| VH CDR2: RIRSKSNNYATYYADSVKD (SEQ ID NO: 19) |
| VH CDR3: GGRGNYLYAMDY (SEQ ID NO: 20) |
| EVQLVESGGGLVQPKGSLKLSCAASGFTFNTYAMYWVCQAPGKGLEW VARIRSKSNNYATYYADSVKDRFTISRDDSQSMLYLQMNNLKTEDTAM YYCVRGGRGNYLYAMDYWGQGTSVTVSS (SEQ ID NO: 35) |
| Light chain variable region |
| VL CDR1: RSSKSLLHSNGNTYLY (SEQ ID NO: 21) |
| VL CDR2: RMSNLAS (SEQ ID NO: 16) |
| VL CDR3: MQHLEYPFT (SEQ ID NO: 17) |
| DIVMTQAAPSVPVTPGESVSISCRSSKSLLHSNGNTYLYWFLQRPGQSPQ LLIYRMSNLASGVPDRFSGSGSGSAFTLRISRVEAEDVGVYYCMQHLEY PFTFGSGTKLEIK (SEQ ID NO: 36) |

The heavy chain constant region of human IgG1: 






 (SEQ ID NO: 37)
Human κ light chain constant region:



 (SEQ ID NO: 38)

Using PEImax 40,000 (Cat #: 24765-1, Polysciences, Inc.), plasmid combinations carrying the heavy chain and light chain coding sequences of the antibodies were transfected into CHO-3E7 cells for the transient expression of anti-CCR8 antibodies. 24 h later, Tryptone N-1 supplement was added to enhance expression/secretion. After shaking culture at 37°C with 5% CO₂ for 6 days, the supernatants were collected, and the antibodies were purified using a protein A agarose gel chromatography column. Then, the purified antibodies were stored in PBS solution for subsequent analytical validation.

### Example 3. In vitro activity identification of chimeric antibodies

### Detection of binding of antibodies to cells overexpressing human CCR8 by flow cytometry

To further investigate the binding ability and binding specificity of the antibodies of the present application for human CCR8, 1 × 10⁵ HEK293/hu-CCR8 cells in 100 µl of medium were incubated with 50 µl of the anti-CCR8 chimeric antibodies of the present application at 4°C in an ELISA plate for 1 h. The antibodies were subjected to 3-fold serial dilution in the cell/antibody mixture, with an initial concentration of 300 nM. Antibodies BMS-986340 and GS-1811 were used as positive controls, and hIgG1 was used as a negative control. After washing the ELISA plate twice with PB, fluorophore (iFluor 647)-labeled goat anti-human IgG (H+L) (Jackson, 109-605-088) was added to each well and then incubated at 4°C for 0.5 h. Then, the samples were analysed by flow cytometry, and an antibody-antigen binding curve was generated, with the mean fluorescence intensity (geometric mean of fluorescence intensity) as the ordinate and the antibody concentration as the abscissa. Raw data were plotted using GraphPad Prism v6.02 software, and EC₅₀ values were determined.

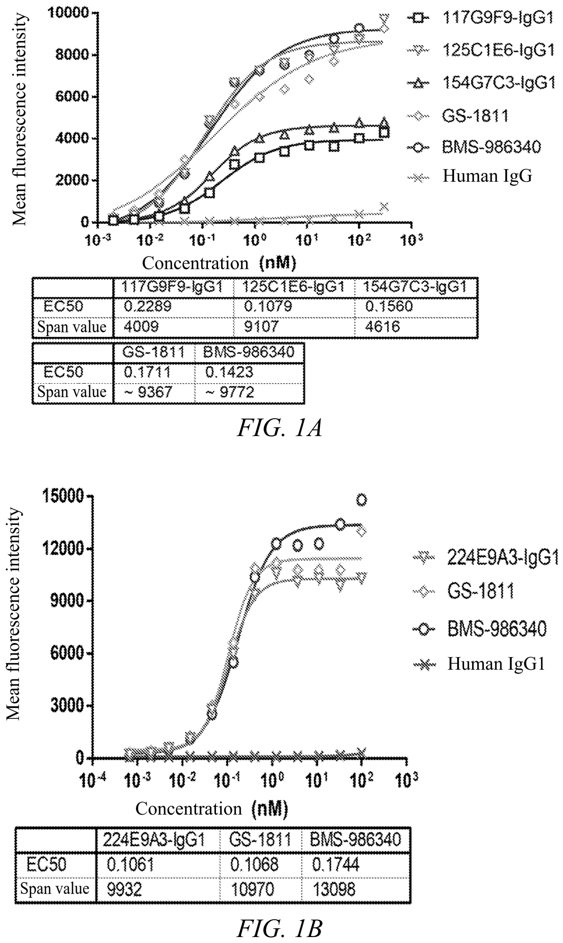

Among these anti-CCR8 chimeric antibodies, as shown in FIGs. 1A and 1B, the monoclonal antibodies of the present application, including 125C1E6, 154G7C3, 117G9F9, and 224E9A3, all bound to cells overexpressing human CCR8, with binding ability comparable to those of the positive controls BMS-986340 and GS-1811.

### Detection of binding of antibodies to cells overexpressing monkey CCR8 by flow cytometry

To further test the cross-reactivity of the anti-CCR8 chimeric antibodies with monkey CCR8, the same method and steps as described above were used, except that HEK293/cyno-CCR8 cells were used instead of HEK293/hu-CCR8 cells.

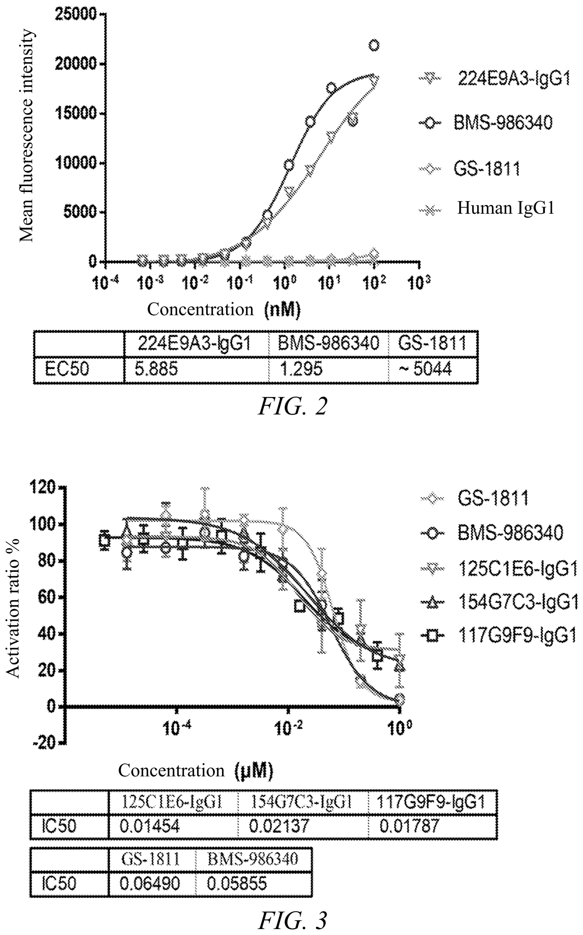

As shown in FIG. 2, the antibody 224E9A3 bound to cells expressing monkey CCR8 with high binding ability comparable to that of BMS-986340, whereas GS-1811 showed no binding to cells expressing monkey CCR8 in FACS detection.

### Analysis of CCR8-CCL1 blockade by detecting CCR8-mediated calcium flux

The binding of the ligand CCL1 to the chemokine receptor CCR8 can induce CCR8-mediated calcium influx. Therefore, the blocking ability of the anti-CCR8 antibodies to CCL1-CCR8 signalling can be determined by measuring cellular calcium flux.

Briefly, 20 µl of CHO-K1/hu-CCR8 cells at a density of 7.5 × 10⁵ cells/ml were seeded into a 384-well assay plate and cultured at 37°C for 16 h. 10 µl of antibody was added to the assay plate, resulting in 5-fold serial dilution of the antibodies in the cell/antibody mixture, with an initial concentration of 1 µM across a total of 8 concentration points, except for 117G9F9, which was subjected to a 5-fold serial dilution in the cell/antibody mixture, with an initial concentration of 0.4 µM across a total of 8 concentration points. Moreover, a working dye solution from the FLIPR^{®} Calcium 4 Assay Kit (Cat #: R7446, Molecular Devices) was added and then incubated at 37°C for 1 h in the dark. BMS-986340 and GS-1811 were used as positive controls. After adding 35 µl of 10 nM recombinant human CCL1 (GenScript, Z02911), the calcium flux fluorescence signal was measured using the FLIPR^{™} TETRA system (Molecular Devices), and data were analysed with GraphPad Prism to obtain IC₅₀ values.

The results are shown in FIG. 3. According to the IC₅₀ values, the anti-CCR8 antibody molecules of the present application exhibited better blocking activity against CCL1-CCR8 signalling compared to the positive controls.

### Antibody-dependent cell-mediated cytotoxicity (ADCC) induced by anti-CCR8 antibodies

The activity of the anti-CCR8 chimeric antibodies in inducing ADCC was evaluated using a reporter gene-based ADCC bioactivity assay.

Briefly, CHO-K1/hu-CCR8 target cells were cultured and harvested, and then seeded into a 96-well plate at a density of 1 × 10⁴ cells/well, with the cells suspended in 40 µl of medium. 20 µl of the antibodies of the present application or positive controls (BMS-986340 and GS-1811) were added to the 96-well plate, resulting in 10-fold serial dilution of the antibodies in the cell/antibody mixture, with an initial concentration of 10 µg/ml (approximately 60 nM) across a total of 7 concentration points. The plate was then incubated at 37°C with 5% CO₂ for 30 min. Subsequently, 40 µl of GS-J2C/CD16A cells (ADCC reporter cell line, Cat#: RD00830, GenScript) at a density of 1.5 × 10⁶ cells/ml, used as effector cells, were added to the 96-well plate and then incubated at 37°C with 5% CO₂ for 6 h. The 96-well plate was taken out, and the activation of the ADCC reporter cell line was analysed using the Bio-Glo^{®} Assay Kit. Luminescence data were detected by PheraStar (BMG) for analysing ADCC reporter gene activation signals, and analysed using GraphPad Prism 6.0.

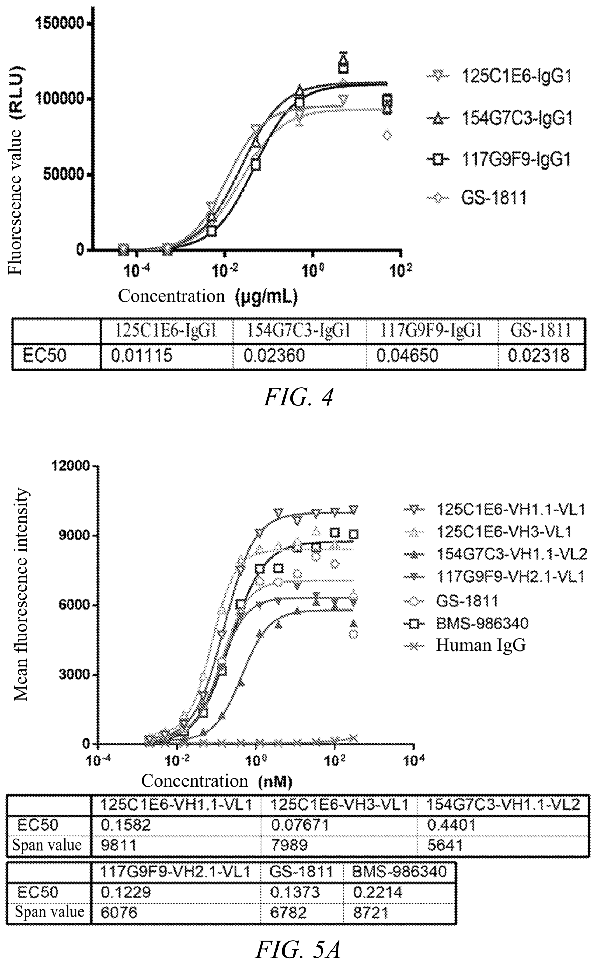

The assay results are shown in FIG. 4. Compared to the positive controls, the anti-CCR8 chimeric antibodies of the present application were all capable of inducing ADCC against CCR8⁺ cells with comparable or higher activity.

### Example 4. Humanization of antibodies

For the humanization of 117G9F9, 125C1E6, and 154G7C3, these murine antibodies were individually aligned with human Ig germline sequences to obtain sequences with the overall best match. The obtained human germline sequences that are highly homologous to the murine antibodies were used as acceptors, and the CDRs of the murine antibodies were grafted into the acceptor frameworks. Then, sequence analysis was performed on the produced antibodies to determine whether there are high-risk sites of potential post-translational modifications in the constructed sequences, including deamidation, isomerization, oxidation, and unpaired cysteine residues that may affect the binding activity and stability of the antibodies.

Referring to the method and steps in Example 2, the DNA sequences encoding the heavy chain and light chain variable regions of the humanized antibodies were synthesized, and then fused to the N-terminus of the coding sequences of the human IgG1 heavy chain constant region and human κ light chain constant region, respectively. These fused sequences were then cloned into the expression plasmid pTT5, downstream of the signal peptide, for secretory expression. The amino acid sequences of the heavy chain and light chain variable regions are listed in Table 2, and the amino acid sequences of the human IgG1 heavy chain constant region and human κ light chain constant region are set forth in SEQ ID NO: 37 and 38, respectively.

**Table 2. Sequence information of humanized antibodies**

| |
|---|
| 125C1E6-VH1.1-VL1 |
| Heavy chain variable region 125C1E6-VH1.1 |
| EVQLVESGGGLVQPGGSLKLSCAASGFNFNAYAMNWVRQASGKGLE WVGRIRSKSNYYATYYADSVKDRFTISRDDSKNTAYLQMNSLKTEDTA VYYCVRQGYFGTRESRRYFDVWGQGTLVTVSS (SEQ ID NO: 24) |
| Light chain variable region 125C1E6-VL1 |
| EIVLTQSPGTLSLSPGERATLSCKASESVGSYVSWYQQKPGQAPRLLIYG ASTRYTGIPARFSGSGSGTDFTLTISSLEPEDFAVYYCGQNYIYPLTFGGG TKLEIK (SEQ ID NO: 25) |
| 125C1E6-VH3-VL1 |
| Heavy chain variable region 125C1E6-VH3 |
| EVQLVESGGGLVQPGRSLRLSCTASGFNFNAYAMNWVRQAPGKGLEW VGRIRSKSNYYATYYADSVKDRFTISRDDSKSIAYLQMNSLKTEDTAVY YCVRQGYFGTRESRRYFDVWGQGTLVTVSS (SEQ ID NO: 26) |
| Light chain variable region 125C1E6-VL1 |
| EIVLTQSPGTLSLSPGERATLSCKASESVGSYVSWYQQKPGQAPRLLIYG ASTRYTGIPARFSGSGSGTDFTLTISSLEPEDFAVYYCGQNYIYPLTFGGG TKLEIK (SEQ ID NO: 25) |
| 154G7C3-VH1.1-VL2 |
| Heavy chain variable region 154G7C3-VH1.1 |
| QVQLVQSGAEVKKPGASVKVSCKVSGYTFTDFEMHWVRQAPGKGLE WIGAFDPETGGTAYNQKFKGRVTMTEDTSTDTAYMELSSLRSEDTAVY YCTGRLRRHFDSWGQGTLVTVSS (SEQ ID NO: 29) |
| Light chain variable region 154G7C3-VL2 |
| DIVMTQSPLSLPVTPGEPASISCRSSQSLVHSYGNTYLHWYLQKPGQSPQ LLIYKVSNRFSGVPDRFSGSGSGTDFTLKISRVEAEDVGVYYCSQSTHVP FTFGQGTKLEIK (SEQ ID NO: 30) |
| 117G9F9-VH2.1-VL1 |
| Heavy chain variable region 117G9F9-VH2.1 |
| EVQLVESGGGLVKPGGSLRLSCAASGFSFNTYAMNWVRQAPGKGLEW VGRIRSKSNYYATYYADSVKDRFTISRDDSKNTLYLQMNSLKTEDTAV YYCVRGGNSYGSSYFDYWGQGTLVTVSS (SEQ ID NO: 33) |
| Light chain variable region 117G9F9-VL1 |
| DIVMTQSPLSLPVTPGEPASISCRSSKSLLHRNGNTYLYWYLQKPGQSPQ LLIYRMSNLASGVPDRFSGSGSGTDFTLKISRVEAEDVGVYYCMQHLEY PFTFGQGTKLEIK (SEQ ID NO: 34) |

### Example 5. Activity characterization of humanized antibodies

### Detection of binding ability of humanized antibodies to cells overexpressing human or monkey CCR8 by flow cytometry

According to the method and steps in Example 3, the binding ability and binding specificity of the humanized antibodies to cells overexpressing human or monkey CCR8, as well as to HEK293 parental cells, were tested.

FIG. 5A shows the antibody-antigen binding curves of the humanized antibodies to cells overexpressing human CCR8. It could be seen that the humanized antibodies of the present disclosure all exhibited relatively high binding ability to cells overexpressing human CCR8. In particular, 125C1E6-VH1.1-VL1 and 125C1E6-VH3-VL1 exhibited higher binding ability than BMS-986340 and GS-1811, which was specifically reflected by higher span values and/or smaller EC₅₀ values.

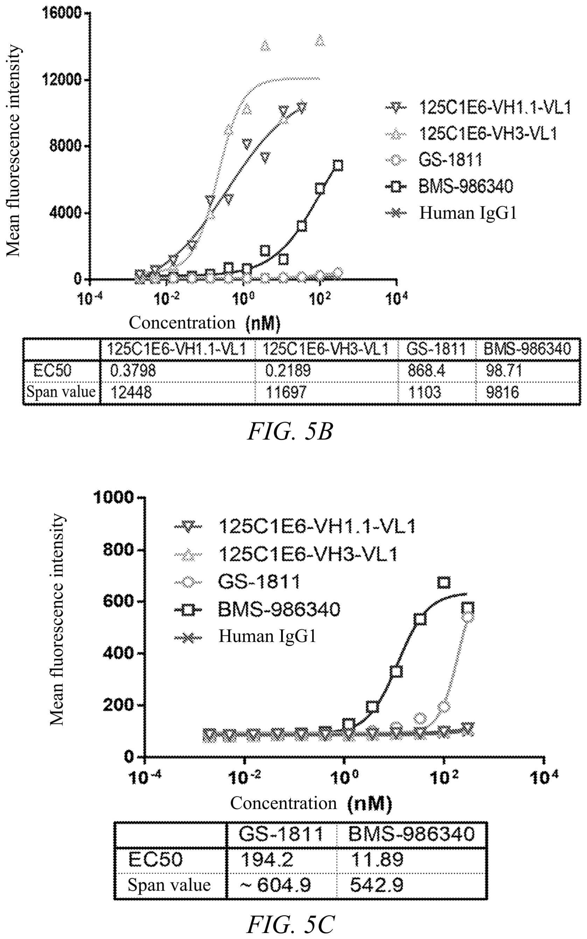

FIG. 5B shows the antibody-antigen binding curves of the humanized antibodies to cells overexpressing monkey CCR8. It can be seen that 125C1E6-VH1.1-VL1 and 125C1E6-VH3-VL1 both bound to cells expressing monkey CCR8 with significantly higher binding ability than BMS-986340, whereas no binding of GS-1811 to monkey CCR8 was detected.

FIG. 5C shows the antibody-antigen binding curves of the humanized antibodies to HEK293 cells. The two positive controls, BMS-986340 and GS-1811, exhibited weak binding to parental HEK293 cells, whereas 125C1E6-VH1.1-VL1 and 125C1E6-VH3-VL1 did not bind to HEK293 cells.

### Antibody-dependent cell-mediated cytotoxicity (ADCC) induced by humanized antibodies

Referring to the method and steps in Example 3, the activity of the humanized antibodies in inducing ADCC was evaluated. In this process, the humanized antibodies and the positive controls were both subjected to 10-fold serial dilution in the cell/antibody mixture with an initial concentration of 10 µg/ml.

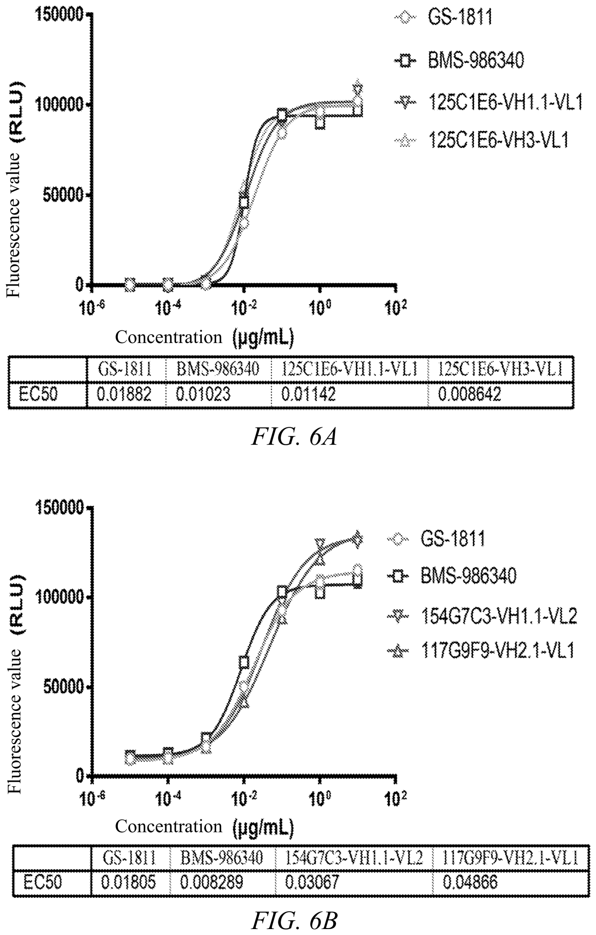

The results are shown in FIGs. 6A and 6B. The humanized anti-CCR8 antibodies all exhibited activity in inducing ADCC against CCR8⁺ cells that is comparable to that of the positive controls.

### Analysis of CCR8-CCL1 blockade by detecting CCR8-mediated calcium flux

Referring to the method and steps in Example 3, the blocking ability of the humanized antibodies to CCL1-CCR8 signalling was determined by measuring cellular calcium flux. In this process, the antibodies were subjected to 5-fold serial dilution in the cell/antibody mixture, with an initial concentration of 1 µM across a total of 8 concentration points.

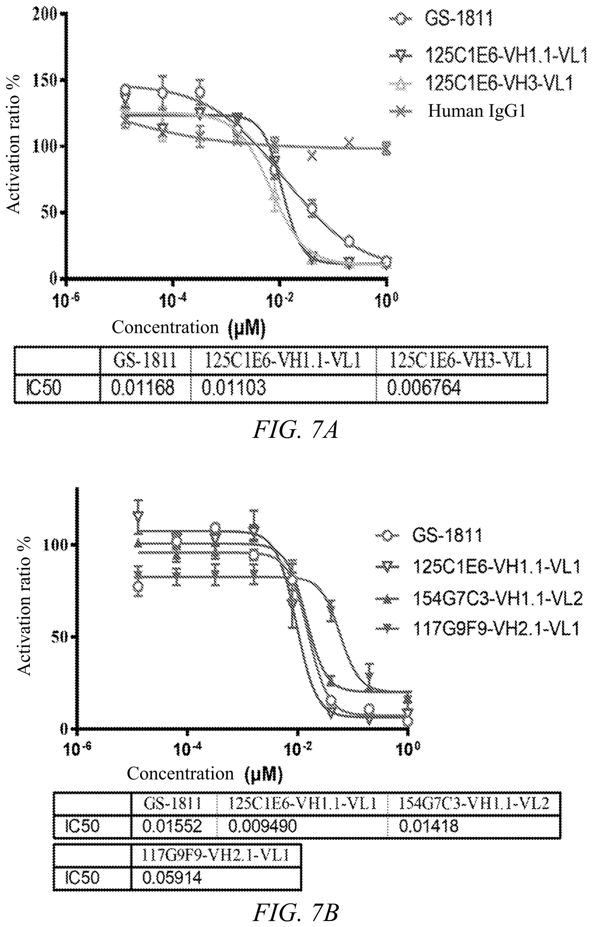

As shown in FIGs. 7A and 7B, 125C1E6-VH1.1-VL1, 125C1E6-VH3-VL1, and 154G7C3-VH1.1-VL2 all exhibited *in vitro* CCR8-CCL1 blocking activity that is comparable to or better than that of the positive control GS-1811.

### Antibody internalization analysis using IncuCyte live-cell analysis system

The internalization of the antibodies was analysed using the Incucyte real-time live-cell system (Sartorius).

Briefly, 50 µl of CHO-K1/hu-CCR8 cells at a density of 2 × 10⁵ cells/ml were seeded into a 96-well assay plate and cultured at 37°C for 16 h. Moreover, the antibodies were labeled prior to the internalization assay. Specifically, the humanized antibodies and Incucyte Fabfluor pH dye were each subjected to 2-fold serial dilution in complete growth medium (90% F12K medium + 10% fetal bovine serum), with an initial concentration of 4 µg/ml across a total of 8 concentration points. Subsequently, the diluted humanized antibodies and Incucyte Fabfluor pH dye were mixed at a volume ratio of 1 : 1, and incubated at 37°C for 15 min in the dark in a 96-well round-bottom plate. 50 µl of the labeled humanized antibodies were added to the 96-well assay plate, and incubated in an Incucyte S3 cell incubator at 37°C for 24 h. The antibody internalization rate was detected by measuring the red fluorescence in CHO-K1/hu-CCR8 cells. BMS-986340 and GS-1811 were used as positive controls, whereas human IgG1 was used as a negative control. Raw data were exported from Incucyte 2022B and analysed using Incucyte 2022B, Microsoft Office Excel 2016, and GraphPad Prism 6. Graphs were plotted with the total fluorescence intensity (RCU × µm²) of each fluorescence image against the antibody concentration.

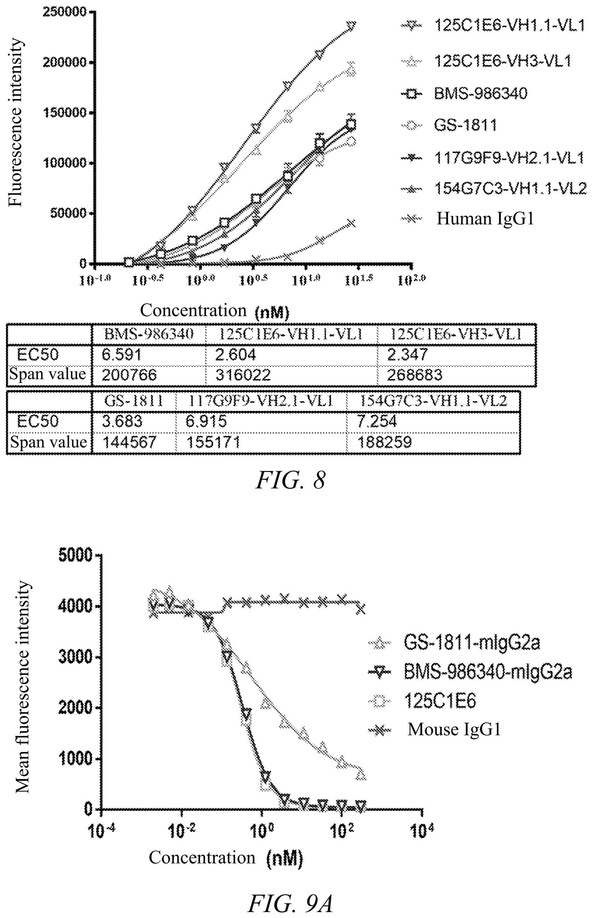

As shown in FIG. 8, the humanized antibodies 125C1E6-VH1.1-VL1 and 125C1E6-VH3-VL1 both exhibited higher internalization activity than the positive controls. In addition, the humanized antibodies 154G7C3-VH1.1-VL2 and 117G9F9-VH2.1-VL1 exhibited comparable internalization activity to the positive controls.

### Example 6. Assay on competitive binding to antigenic epitope

To determine whether the humanized antibodies bind to the same or similar epitopes as the positive controls, the competitive binding of the antibodies to HEK293/hu-CCR8 cells was assayed by FACS.

Briefly, the 125C1E6-VH3-VL1 antibody containing hIgG1-Fc (i.e., SEQ ID NO: 37) at a final concentration of 0.45 nM was first incubated with 1 × 10⁵ HEK293/hu-CCR8 cells at 4°C for 0.2 h in a total volume of 100 µl. Subsequently, 50 µl of antibodies containing mouse Fc were added, including BMS-986340-mIgG2a (with the heavy chain constant region of BMS-986340 replaced by the mouse IgG2a heavy chain constant region of SEQ ID NO: 39 and the light chain constant region replaced by SEQ ID NO: 40), GS-1811-mIgG2a (with the heavy chain constant region of GS-1811-mIgG2a replaced by SEQ ID NO: 39 and the light chain constant region replaced by SEQ ID NO: 40), and 125C1E6-mIgG2c (purified from hybridoma supernatant), or mouse IgG1. The antibodies were subjected to 3-fold serial dilution in the cell/antibody mixture with an initial concentration of 300 nM, and then incubated at 4°C for 0.8 h. Then, 125C1E6-VH3-VL1 bound to the cell surface was detected using fluorophore (iFluor 647)-labeled goat anti-human IgG (H+L). Data were analysed using GraphPad Prism 6.0. The results are shown in FIG. 9A.

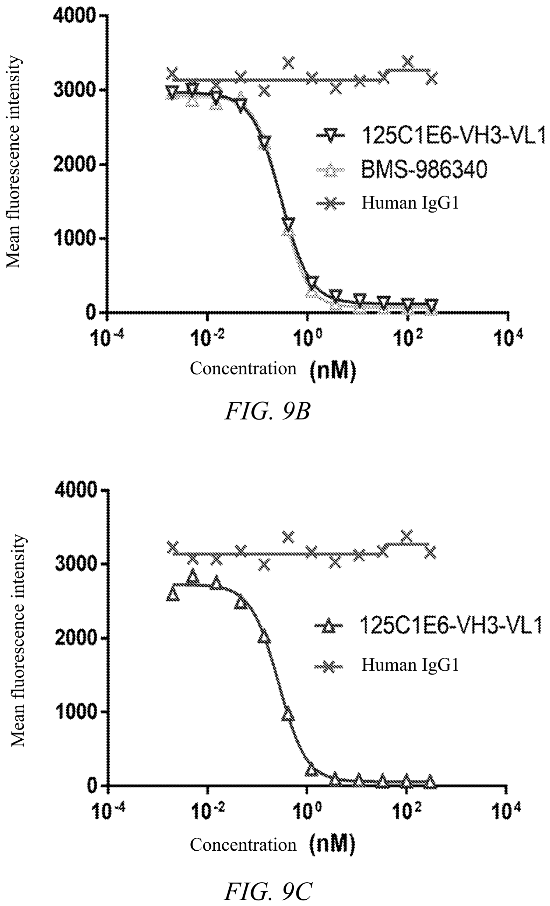

Similarly, to further determine the antigenic epitope competition among several antibodies, BMS-986340-mIgG2a at a final concentration of 0.28 nM was first added, followed by the addition of the competitive antibody 125C1E6-VH3-VL1, BMS-986340, or human IgG1; alternatively, GS-1811-mIgG2a at a final concentration of 0.28 nM was first added, followed by the addition of 125C1E6-VH3-VL1 or human IgG1. Other conditions were the same as above. The competitive antibodies were subjected to 3-fold serial dilution in the cell/antibody mixture at an initial concentration of 300 nM. BMS-986340-mIgG2a or GS-1811-mIgG2a bound to the cell surface was detected using fluorophore (iFluor 647)-labeled goat anti-mouse IgG (H+L). The results are shown in FIGs. 9B and 9C.
Heavy chain constant region of mIgG2a: 
AKTTAPSVYPLAPVCGDTTGSSVTLGCLVKGYFPEPVTLTWNSGSLSSGVH
TFPAVLQSDLYTLSSSVTVTSSTWPSQSITCNVAHPASSTKVDKKIEPRGPTI
KPCPPCKCPAPNLLGGPSVFIFPPKIKDVLMISLSPIVTCVVVDVSEDDPDVQ
ISWFVNNVEVHTAQTQTHREDYNSTLRVVSALPIQHQDWMSGKEFKCKV
NNKDLPAPIERTISKPKGSVRAPQVYVLPPPEEEMTKKQVTLTCMVTDFMP
EDIYVEWTNNGKTELNYKNTEPVLDSDGSYFMYSKLRVEKKNWVERNSY
SCSVVHEGLHNHHTTKSFSRTPGK (SEQ ID NO: 39)
Light chain constant region:

RADAAPTVSIFPPSSEQLTSGGASVVCFLNNFYPKDINVKWKIDGSERQNG
VLNSWTDQDSKDSTYSMSSTLTLTKDEYERHNSYTCEATHKTSTSPIVKSF
NRNEC (SEQ ID NO: 40)

As shown in FIG. 9A, BMS-986340-mIgG2a and GS-1811-mIgG2a were both capable of competing with 125C1E6-VH3-VL1 for the CCR8 binding site. The results shown in FIGs. 9B and 9C are consistent with those in FIG. 8A. It could be seen that the antigenic epitope bound by the 125C1E6 antibody overlapped with the CCR8 epitopes targeted by the two positive controls.

Although the present application has been described in conjunction with one or more embodiments, it should be understood that the present application is not limited to these embodiments. The descriptions in the present application are intended to encompass all variations and equivalents, which are all included within the spirit and scope of the appended claims. All literatures cited herein are incorporated herein by reference in their entireties.

## Claims

1. An isolated monoclonal antibody or an antigen-binding portion thereof capable of specifically binding to CCR8, **characterized in that** the isolated monoclonal antibody or the antigen-binding portion thereof comprises:
i) a heavy chain variable region, comprising VH CDR1, VH CDR2, and VH CDR3, wherein the VH CDR1, the VH CDR2, and the VH CDR3 respectively comprise amino acid sequences as set forth in: (1) SEQ ID NOs: 1, 2, and 3; (2) SEQ ID NOs: 7, 8, and 9; (3) SEQ ID NOs: 13, 2, and 14; or (4) SEQ ID NOs: 18, 19, and 20, or comprise amino acid sequences having 1-3 amino acid substitutions in each CDR compared to the amino acid sequences described above; and/or
ii) a light chain variable region, comprising VL CDR1, VL CDR2, and VL CDR3, wherein the VL CDR1, the VL CDR2, and the VL CDR3 respectively comprise amino acid sequences as set forth in: (1) SEQ ID NOs: 4, 5, and 6; (2) SEQ ID NOs: 10, 11, and 12; (3) SEQ ID NOs: 15, 16, and 17; or (4) SEQ ID NOs: 21, 16, and 17, or comprise amino acid sequences having 1-3 amino acid substitutions in each CDR compared to the amino acid sequences described above.

2. The isolated monoclonal antibody or the antigen-binding portion thereof according to claim 1, **characterized in that** the isolated monoclonal antibody or the antigen-binding portion thereof comprises a heavy chain variable region and a light chain variable region, wherein the VH CDR1, the VH CDR2, the VH CDR3, the VL CDR1, the VL CDR2, and the VL CDR3 respectively comprise amino acid sequences as set forth in: (1) SEQ ID NOs: 1, 2, 3, 4, 5, and 6; (2) SEQ ID NOs: 7, 8, 9, 10, 11, and 12; (3) SEQ ID NOs: 13, 2, 14, 15, 16, and 17; or (4) SEQ ID NOs: 18, 19, 20, 21, 16, and 17.

3. The isolated monoclonal antibody or the antigen-binding portion thereof according to claim 1 or 2, **characterized in that** the heavy chain variable region comprises an amino acid sequence having at least 80%, 85%, 90%, 91%, 92%, 93%, 94%, 95%, 96%, 97%, 98%, 99%, or 100% sequence identity to SEQ ID NO: 22, 24, 26, 27, 29, 31, 33, or 35.

4. The isolated monoclonal antibody or the antigen-binding portion thereof according to any one of claims 1 to 3, **characterized in that** the light chain variable region comprises an amino acid sequence having at least 80%, 85%, 90%, 91%, 92%, 93%, 94%, 95%, 96%, 97%, 98%, 99%, or 100% sequence identity to SEQ ID NO: 23, 25, 28, 30, 32, 34, or 36.

5. The isolated monoclonal antibody or the antigen-binding portion thereof according to any one of claims 1 to 4, **characterized in that** the isolated monoclonal antibody or the antigen-binding portion thereof comprises a heavy chain variable region and a light chain variable region, wherein the heavy chain variable region and the light chain variable region respectively comprise amino acid sequences having at least 80%, 85%, 90%, 91%, 92%, 93%, 94%, 95%, 96%, 97%, 98%, 99%, or 100% sequence identity to (1) SEQ ID NOs: 22 and 23;(2) SEQ ID NOs: 24 and 25; (3) SEQ ID NOs: 26 and 25; (4) SEQ ID NOs: 27 and 28; (5) SEQ ID NOs: 29 and 30; (6) SEQ ID NOs: 31 and 32; (7) SEQ ID NOs: 33 and 34; or (8) SEQ ID NOs: 35 and 36.

6. The isolated monoclonal antibody or the antigen-binding portion thereof according to any one of claims 1 to 5, **characterized in that** the isolated monoclonal antibody or the antigen-binding portion thereof further comprises a heavy chain constant region and/or a light chain constant region.

7. The isolated monoclonal antibody or the antigen-binding portion thereof according to claim 6, **characterized in that** the heavy chain constant region is an IgG1 heavy chain constant region.

8. The isolated monoclonal antibody or the antigen-binding portion thereof according to claim 7, **characterized in that** the heavy chain variable region is a human IgG1 heavy chain constant region comprising an amino acid sequence as set forth in SEQ ID NO: 37.

9. The isolated monoclonal antibody or the antigen-binding portion thereof according to claim 6, **characterized in that** the light chain constant region is a κ light chain constant region.

10. The isolated monoclonal antibody or the antigen-binding portion thereof according to claim 9, **characterized in that** the light chain constant region is a human κ light chain constant region comprising an amino acid sequence as set forth in SEQ ID NO: 38.

11. The isolated monoclonal antibody or the antigen-binding portion thereof according to any one of claims 1 to 10, **characterized in that** the isolated monoclonal antibody or the antigen-binding portion thereof is murine, chimeric, or humanized.

12. The isolated monoclonal antibody or the antigen-binding portion thereof according to any one of claims 1 to 11, **characterized in that** the isolated monoclonal antibody or the antigen-binding portion thereof i) is capable of binding to human CCR8, ii) is capable of blocking CCR8-CCL1 binding/interaction, iii) is capable of be internalized by CCR8⁺ cells, vi) is capable of inducing antibody-dependent cell-mediated cytotoxicity against CCR8⁺ cells, and/or v) has an *in vivo* anti-tumour effect.

13. An immunoconjugate, **characterized in that** the immunoconjugate comprises i) the antibody or the antigen-binding portion thereof according to any one of claims 1 to 12, and ii) a cytotoxic molecule, wherein i) and ii) are linked via a linker or directly.

14. A bispecific molecule, **characterized in that** the bispecific molecule comprises i) the antibody or the antigen-binding portion thereof according to any one of claims 1 to 12, and ii) a second functional group, wherein i) and ii) bind to different antigens or different epitopes of a same antigen, and i) and ii) are linked.

15. A chimeric antigen receptor, **characterized in that** the chimeric antigen receptor comprises i) an extracellular antigen-binding domain, ii) a transmembrane domain, and iii) an intracellular signal transduction domain, wherein the extracellular antigen-binding domain comprises the antibody or the antigen-binding portion thereof according to any one of claims 1 to 12.

16. An immune cell, **characterized in that** the immune cell comprises the chimeric antigen receptor according to claim 15.

17. An oncolytic virus, **characterized in that** the oncolytic virus expresses the antibody or the antigen-binding portion thereof according to any one of claims 1 to 12.

18. A nucleic acid molecule, **characterized in that** the nucleic acid molecule encodes the antibody or the antigen-binding portion thereof according to any one of claims 1 to 12, the immunoconjugate according to claim 13, the bispecific molecule according to claim 14, or the chimeric antigen receptor according to claim 15.

19. An expression vector, **characterized in that** the expression vector comprises the nucleic acid molecule according to claim 18.

20. A host cell, **characterized in that** the host cell comprises the expression vector according to claim 19 or having the nucleic acid molecule according to claim 18 integrated into a genome thereof.

21. A composition, **characterized in that** the composition comprises the antibody or the antigen-binding portion thereof according to any one of claims 1 to 12, the immunoconjugate according to claim 13, the bispecific molecule according to claim 14, the chimeric antigen receptor according to claim 15, the immune cell according to claim 16, the oncolytic virus according to claim 17, the nucleic acid molecule according to claim 18, the expression vector according to claim 19, or the host cell according to claim 20.

22. The composition according to claim 21, **characterized in that** the composition is a pharmaceutical composition further comprising a pharmaceutically acceptable carrier.

23. The composition according to claim 21 or 22, **characterized in that** the composition further comprises a PD-1 antibody.

24. The composition according to any one of claims 21 to 23 for use in the preparation of a medicament for treating or alleviating CCR8-related cancer.

25. The use according to claim 24, **characterized in that** the CCR8-related cancer is a solid tumour.

26. The use according to claim 25, **characterized in that** the solid tumour is non-small cell lung cancer (NSCLC), head and neck squamous cell carcinoma (HNSCC), gastric cancer, microsatellite-stable colorectal cancer, or cervical cancer.

27. The use according to claim 24, **characterized in that** the CCR8-related cancer is T-cell lymphoma.

28. A method for preparing a CCR8 antibody, **characterized in that** the method comprises: i) administering to an animal body a DNA molecule capable of expressing a CCR8 protein, an RNA molecule capable of expressing a CCR8 protein, and/or a cell capable of overexpressing a CCR8 protein; ii) collecting lymph node cells and/or spleen cells from the animal body; and iii) fusing the lymph node cells and/or the spleen cells with myeloma cells to produce hybridomas.

29. The method according to claim 28, **characterized in that** i) administering to an animal body an RNA molecule capable of expressing a CCR8 protein; ii) collecting lymph node cells and/or spleen cells from the animal body; and iii) fusing the lymph node cells and/or the spleen cells with myeloma cells to produce hybridomas.
